(19) Europäisches Patentamt / European Patent Office / Office européen des brevets

(11) **EP 3 100 023 B1**

(12) **EUROPEAN PATENT SPECIFICATION**

(45) Date of publication and mention of the grant of the patent:
**08.04.2020 Bulletin 2020/15**

(21) Application number: **15743662.7**

(22) Date of filing: **29.01.2015**

(51) Int Cl.:
***G01N 1/00*** *(2006.01)*

(86) International application number:
**PCT/US2015/013607**

(87) International publication number:
**WO 2015/116862 (06.08.2015 Gazette 2015/31)**

(54) **METHODS FOR ORGANIC SOLUTE SAMPLING OF AQUEOUS AND HETEROGENEOUS ENVIRONMENTS**

VERFAHREN ZUR PROBENAHME ORGANISCHER GELÖSTER STOFFE AUS WÄSSRIGEN UND HETEROGENEN UMGEBUNGEN

PROCÉDÉS POUR L'ÉCHANTILLONNAGE DE SOLUTÉ ORGANIQUE DANS DES ENVIRONNEMENTS AQUEUX ET HÉTÉROGÈNES

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **29.01.2014 US 201461932890 P**

(43) Date of publication of application:
**07.12.2016 Bulletin 2016/49**

(73) Proprietor: **University of Maryland, Baltimore Baltimore, MD 21201 (US)**

(72) Inventors:
• **MACKERELL, Alexander, D., Jr. Baltimore, MD (US)**
• **RAMAN, E., Prabhu San Diego, CA 92126 (US)**
• **LAKKARAJU, Sirish, Kaushik Baltimore, MD (US)**

(74) Representative: **Hoffmann Eitle Patent- und Rechtsanwälte PartmbB Arabellastraße 30 81925 München (DE)**

(56) References cited:
WO-A1-2005/114458    US-A1- 2004 267 509
US-A1- 2005 123 993    US-A1- 2008 113 410

• YUQING DENG ET AL: "Computation of binding free energy with molecular dynamics and grand canonical Monte Carlo simulations", JOURNAL OF CHEMICAL PHYSICS, vol. 128, no. 11, 21 March 2008 (2008-03-21), page 115103, XP055387115, US ISSN: 0021-9606, DOI: 10.1063/1.2842080

• MATTHEW CLARK ET AL: "Grand Canonical Free-Energy Calculations of Protein-Ligand Binding", JOURNAL OF CHEMICAL INFORMATION AND MODELING, vol. 49, no. 4, 27 April 2009 (2009-04-27) , pages 934-943, XP055387261, US ISSN: 1549-9596, DOI: 10.1021/ci8004397

• ATTILA MALASICS ET AL: "Simulating prescribed particle densities in the grand canonical ensemble using iterative algorithms", JOURNAL OF CHEMICAL PHYSICS, vol. 128, no. 12, 28 March 2008 (2008-03-28), page 124102, XP055387165, US ISSN: 0021-9606, DOI: 10.1063/1.2839302

• SIRISH KAUSHIK LAKKARAJU ET AL: "Sampling of Organic Solutes in Aqueous and Heterogeneous Environments Using Oscillating Excess Chemical Potentials in Grand Canonical-like Monte Carlo-Molecular Dynamics Simulations", JOURNAL OF CHEMICAL THEORY AND COMPUTATION: JCTC, vol. 10, no. 6, 10 June 2014 (2014-06-10), pages 2281-2290, XP055387254, US ISSN: 1549-9618, DOI: 10.1021/ct500201y

**(Cont. next page)**

- VITALIS, A ET AL.: 'ISIM: A Program for Grand Canonical Monte Carlo Simulations of the Ionic Environment of Biomolecules.' JOURNAL: MOLECULAR SIMULATION, [Online] vol. 30, no. ISSUE, 15 January 2004, pages 45 - 61, XP008096890 Retrieved from the Internet: <URL:http://www.tandfonline.com/doi/abs/10.1080/08927020310001597862>

- SUN, L ET AL.: 'Monte Carlo Simulations of an Isolated n-Octadecane Chain Solvated in Water?Acetonitrile Mixtures.' JOURNAL OF CHEMICAL THEORY AND COMPUTATION, [Online] vol. 3, no. 2, March 2007, pages 350 - 357, XP055215348 Retrieved from the Internet: <URL:http://pubs.acs.org/doi/abs/10.1021/ct600239z >DOI:10.1021/ct600239z> [retrieved on 2015-03-25]

**Description**

STATEMENT OF FEDERALLY SPONSORED RESEARCH AND DEVELOPMENT

This invention was made in part by government support under grant number CA107331 awarded by the National Institutes of Health. The United States Government has certain rights in the invention.

FIELD OF THE INVENTION

The present invention generally relates to computer assisted methods for using organic solutes to sample aqueous and heterogeneous environments. More specifically, it relates to computer assisted methods for determining the spatial distributions and thermodynamics of small molecules in aqueous environments and heterogenous environments, including heterogenous environments containing a protein having deep or occluded binding sites. The methods provided herein may be used for thermodynamic studies of complex aqueous systems and for computer-aided drug design.

BACKGROUND

Chemical potential ($\mu$) describes the equilibrium movement of particles between two phases or states. The driving force behind this movement comes from 1) a concentration gradient, namely, particles tend to move from a region of higher concentration to a region of lower concentration to gain mixing entropy; and 2) chemical affinity: particles are attracted to regions of high chemical affinity (See Dill KA & Bromberg S (2003), Chemistry and Biology).

Excess chemical potential ($\mu_{ex}$) is the quasistatic work to bring a particle (e.g. solute molecule) from the gas phase to the solvent; $\mu_{ex} = \mu - \mu_{id}$, where $\mu$ and $\mu_{id}$ are the chemical potential and the ideal gas chemical potential of the solute, respectively. In the context of statistical mechanics, chemical potential allows for the thermodynamic state of a system to be defined in terms of a grand canonical (GC) ensemble ($\mu VT$) that allows for variation in the species concentrations across phases/states. Simulation procedures have long evolved towards efficiently determining Gibbs' free energy of hydration (HFE), chemical affinity and other thermodynamically relevant properties of water and other small solute molecules from GC ensembles instead of the more conventional isothermal, isobaric (NPT), canonical (NVT) or microcanonical (NVE) ensembles where the concentration of the species is fixed. To date, many of the GC ensemble strategies have employed Monte-Carlo (MC) simulations to either drive the sampling of water molecules or individual small molecules around proteins or crystal environments, or alternately to improve the accuracy of relative HFE calculations in free energy perturbation (FEP) calculations. However, since Grand Canonical Monte Carlo (GCMC) simulations of systems containing explicit solvent to represent the bulk-phase suffer from convergence problems due to low acceptance rates encountered for the insertion of solutes, simulations in the past were restricted towards investigation of the chemical affinities of only the solvent, simulations investigating the chemical affinities to drive individual solute sampling in the absence of explicit solvent, determine the thermodynamic properties in crystal conditions, or simulations to investigate the use expanded ensemble strategies.

Deng Y and Roux B (J Chem Phys. 2008 Mar 21; 128(11): 115103) describe a computation of binding free energy with molecular dynamics and grand canonical Monte Carlo simulations. Clark M et al. (J Chem Inf Model. 2009 Apr; 49(4): 934-43) describe grand canonical free-energy calculations of protein-ligand binding.

In the context of protein and macromolecular environments, chemical fragment sampling simulation techniques have been employed for discovery or rational design of molecules that can bind to macromolecular targets with high affinities so as to achieve a desired biological outcome. The Site Identification by Ligand Competitive Saturation (SILCS) method is one such technique that identifies the location and approximate affinities of different functional groups on a target macromolecular surface by performing Molecular Dynamics (MD) simulations of the target in an aqueous solution of solute molecules that are representative of different chemical fragments. However, these MD isothermal, isobaric (NPT) ensembles suffer from the long diffusion time scales of the solutes through explicit solvent and macromolecule environments, especially when the macromolecular binding sites are deeply buried and inaccessible to the solvent (i.e. occluded). These limitations imply that only binding sites on a protein that are accessible to the bulk solvent can be studied. However, a large number of biologically important proteins such as the G-protein coupled receptors (GPCRs), nuclear receptor proteins as well as other macromolecules, have deep or occluded ligand binding pockets, and simulations to study the affinity of small molecules for these occluded pockets are generally time intensive.

The methods of the present invention overcome these limitations, yielding accurate spatial distributions for solutes in aqueous macromolecular environments.

## SUMMARY OF THE INVENTION

The present invention is defined as follows:

[1] A computational method for sampling the spatial distribution of one or more solutes and water in a defined region of space (system) comprising:

1) assigning a target concentration, $N_{tgt}$, of each of the one or more solutes and water;
2) sampling the spatial distribution of the one or more solutes and water in a computationally defined region of space using Grand-Canonical Monte-Carlo (GCMC) Metropolis sampling criteria, wherein the excess chemical potential ($\mu_{ex}$) assigned for each of the one or more solutes, if present, and water is set to 0, if present;
3) updating $\mu_{ex}$ of each of the one or more solutes and water from the difference in current concentration in the defined region of space ($N_{sys}$) and the target ($N_{tgt}$),.
4) repeating steps 2) and 3) using the updated values of $\mu_{ex}$ in step 2) to obtain a spatial distribution of the one or more solutes and water;

wherein the system further comprises one or more macromolecules, wherein the spatial distribution of one or more solutes or water is used to identify preferential affinity of each of the solutes or water to each of the one or more macromolecules, and wherein said one or more macromolecules are selected from a protein, RNA, DNA, carbohydrate, lipid, organic chemical, inorganic chemical, or a combination thereof.

[2] The method according to [1] wherein the system contains water and one solute and where the target concentration of the solute and water is set at 1 M and 55 M, respectively, from which the hydration free energy of the solute is obtained from the value of $\mu_{ex}$

[3] The method according to [1], wherein the $\mu_{ex}$ of one or more solutes and water is alternately increased and decreased during the GCMC operations across consecutive cycles involving steps 2)-4), after the concentration of the solutes and the water reach their target value.

[4] The method according to [1], wherein, the proportion of GCMC steps for each of the one or more solutes and water is assigned based on the target concentration of each of the solutes and water.

[5] The method according to [1], wherein the system containing the solutes and or water is encompassed in a larger system containing said solutes and or water.

[6] The method according to [5], wherein the system additionally includes one or more macromolecules.

[7] The method according to [1], wherein the spatial distribution following step 2) is sampled with a molecular dynamics simulation.

[8] The method according to [1], wherein $\mu_{ex}$ is increased or decreased equal to $N_{tgt}/N_{sys}$.

[9] The method according to [1], wherein $\mu_{ex}$ is increased when $N_{sys}$ is lower than the $N_{tgt}$ and decreased when $N_{sys}$ is greater than $N_{tar}$.

[10] The method according to [1], wherein said computationally defined region comprises an inner region containing the one or more solutes and water, located within a larger outer region containing additional water.

[11] The method according to [1], wherein an output of the spatial distributions of the one or more solutes and water is generated.

## BRIEF DESCRIPTION OF DRAWINGS

Non-limiting exemplary embodiments are described herein, with reference to the following figures:

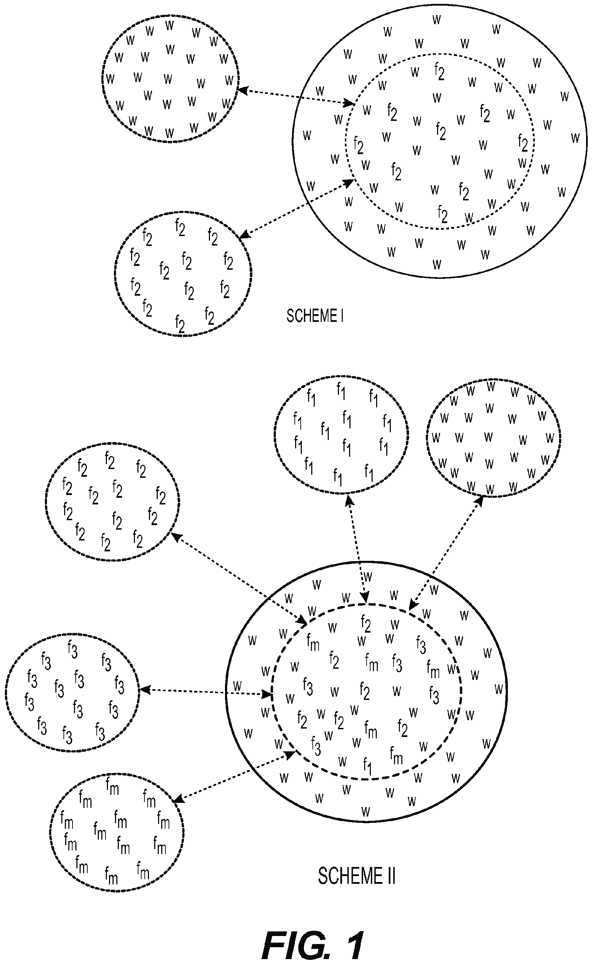

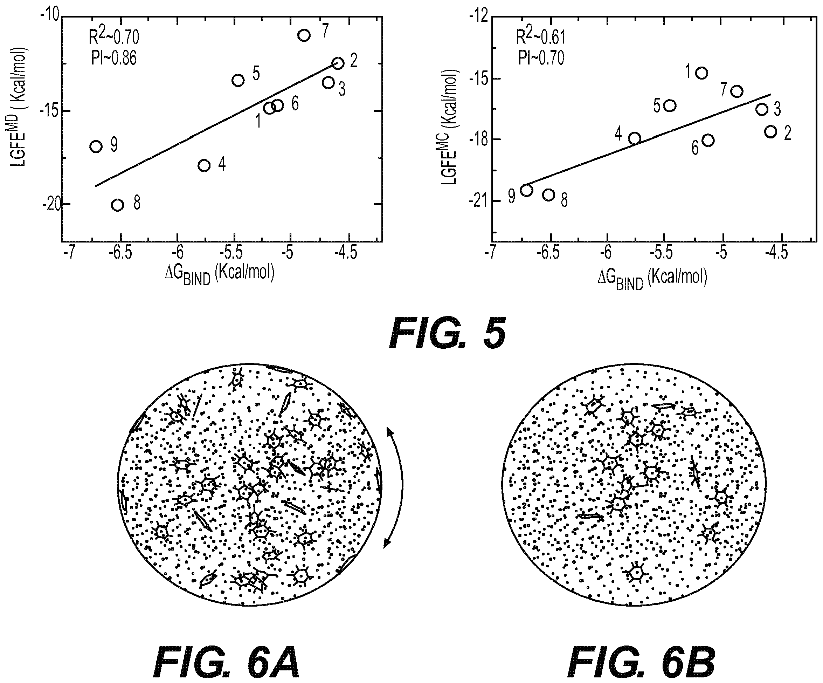

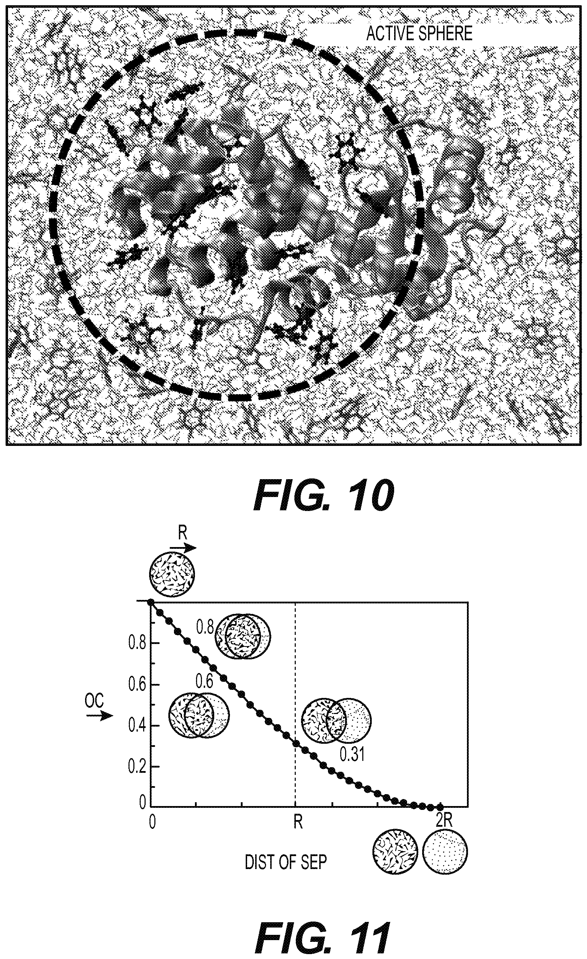

FIG. 1 illustrates an example setup for the standard state (Scheme I) and the aqueous solute mixture (Scheme II) GCMC-MD simulations. Water and the solute molecules are exchanged between their respective reservoirs and the spherical simulation systems, A, indicated by the dashed boundary, defined by radius $r_A$. System A is immersed in a larger system, B defined by the solid boundary, that included additional waters of radius $r_B$ set to $r_A$+ 5 Å in the present study to prevent hydrophobic solutes from occupying the edge of the system A (see Figure 6). Alternatively, the larger system in which system A is immersed may be treated using periodic boundary conditions and include other chemical entities in addition to water (see Fig. 10).

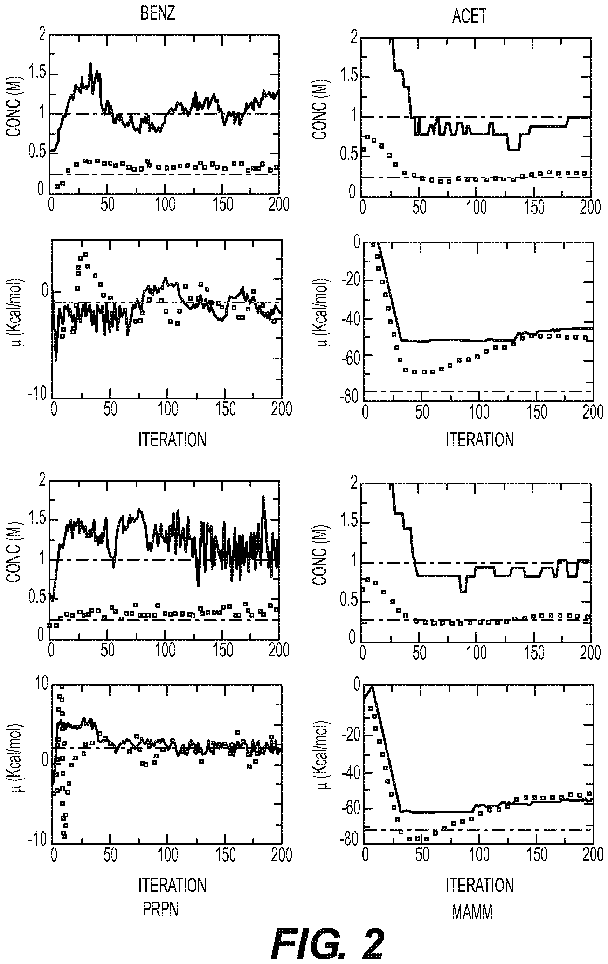

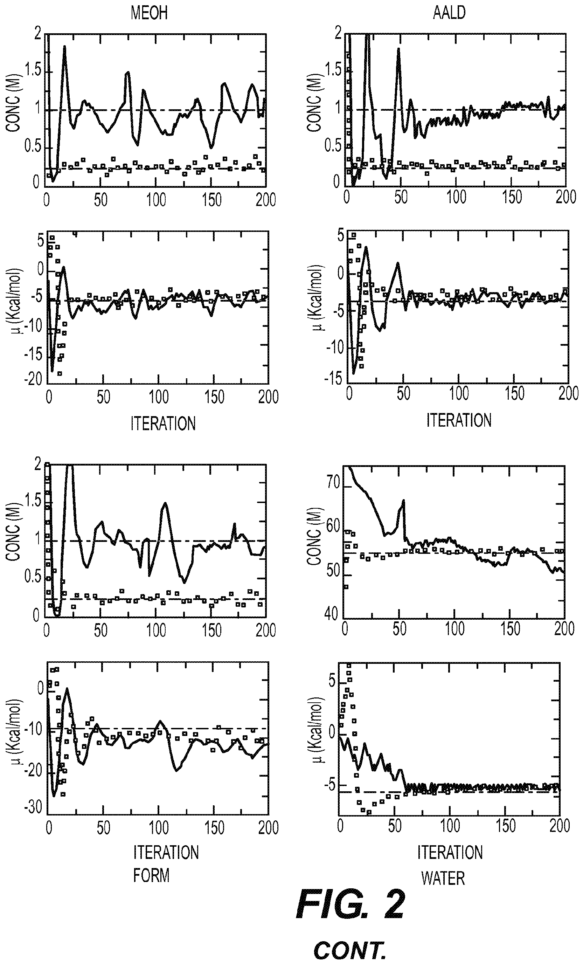

FIG. 2 depicts $\mu_{ex}$ and concentrations of the solutes and the water as a function of the GCMC-MD iterations. $\mu_{ex}$ for the solutes and the water was varied every iteration based on their respective concentrations in the simulation system A. The target concentration of the solutes was 1 M and 0.25 M in Scheme I (black) and Scheme II (dotted line) respectively, while water was maintained at bulk concentration of 55 M in both the systems. Solutes are benzene (BENZ), propane (PRPN), acetate (ACET), methylammonium (MAMM), methanol (MEOH), formamide(FORM) and acetaldehyde (AALD).

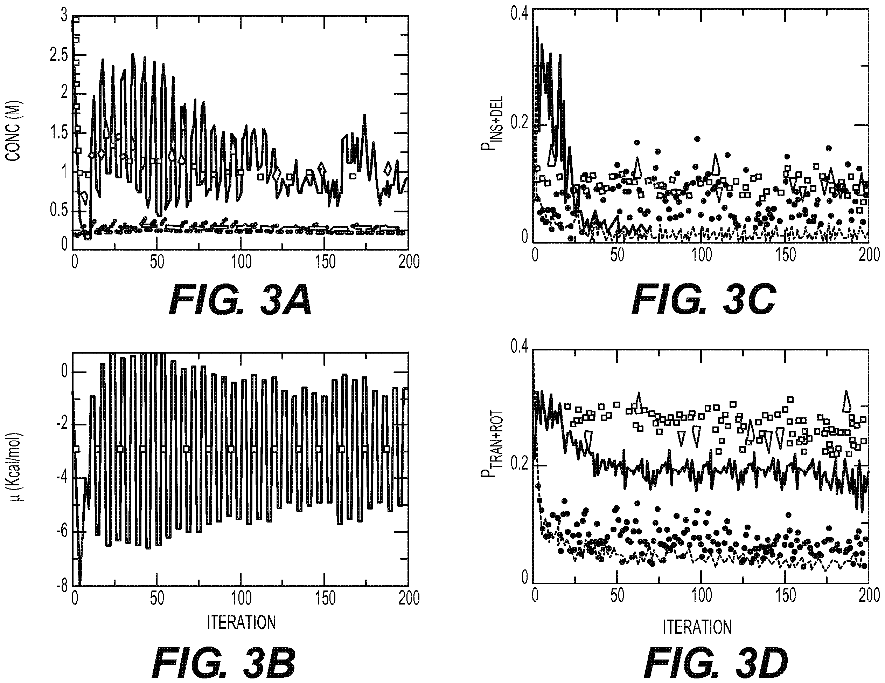

FIG. 3 shows: (A) Concentration; (B) $\mu_{ex}$ of acetaldehyde; and the average probabilities of (C) insertion+deletion ($P_{ins+del}$) and (D) translation+rotation ($P_{trans+rot}$) as a function of the number of GCMC-MD iterations from the Scheme I & II GCMC-MD of acetaldehyde with $\mu_{ex}$ fixed at the HFE (black squares, hash line) or fluctuated by $d\mu_{ex}$ (black line, open circles) respectively. Note that the number of solute exchanges with the gas-phase reservoir are greater with the fluctuating $\mu_{ex}$ while the average concentration and $\mu_{ex}$ are approximately equivalent.

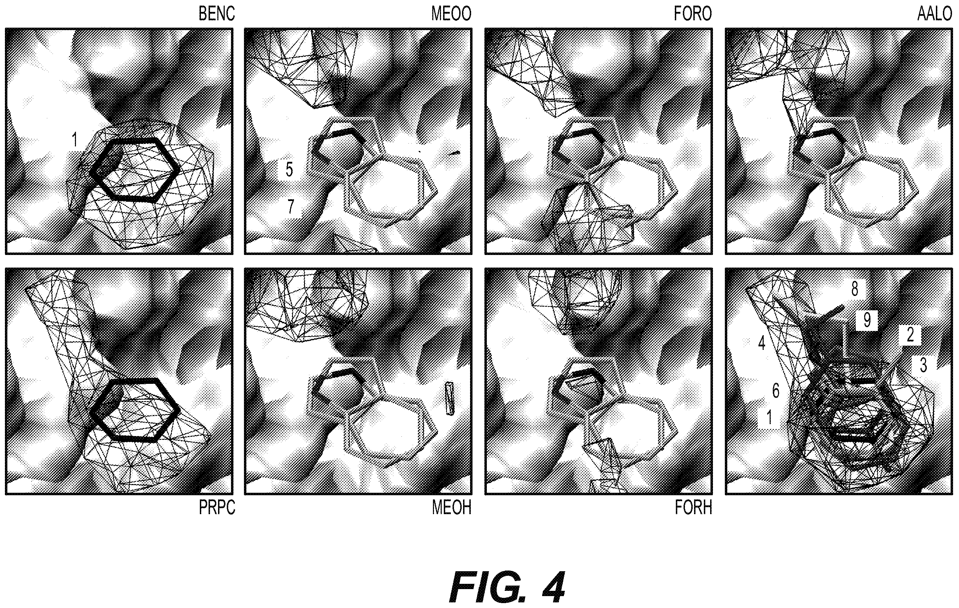

FIG. 4 illustrates selected GFE Fragmaps at the ligand binding site of the T4-lysozyme L99A from a 10×50 ns GCMC-MD simulation and the minimized crystal conformations of the 9 ligands (as further described below); protein atoms occluding the view of the binding pocket were removed for clear visualization. FragMaps are displayed at a cutoff of -1.2 kcal/mol for the BENC (aromatic carbons from BENZ), and PRPC (aliphatic carbons from PRPN) and FragMaps are displayed at a cutoff of -0.5 kcal/mol for AALO, MEOO, FORO (polar acceptor oxygens from AALD, MEOH, and FORH, respectively) and MEOH, and FORH groups (polar donor hydrogens from MEOH and FORH, respectively) groups.

FIG. 5 shows the correlation of the experimental binding affinity ΔG° (from Morton A, Baase WA, & Matthews BW (1995), Biochemistry 34(27):8564-8575) with the LGFE scores for the nine ligands considered (as further described below). The LGFE scores are obtained from MD and MC conformational ensembles of the ligands (LGFE$^{MD}$, LGFE$^{MC}$) and the GFE FragMaps. Overall maps have a very good correlation (high $R^2$ and predictive index, PI).

FIG. 6 shows distribution of benzene in a spherical boundary aqueous system. When solute and waters share the same spherical boundary, hydrophobic solutes accumulate at the edge of the wall. B. To avoid this, the system A is immersed in a larger system B as described in the main text.

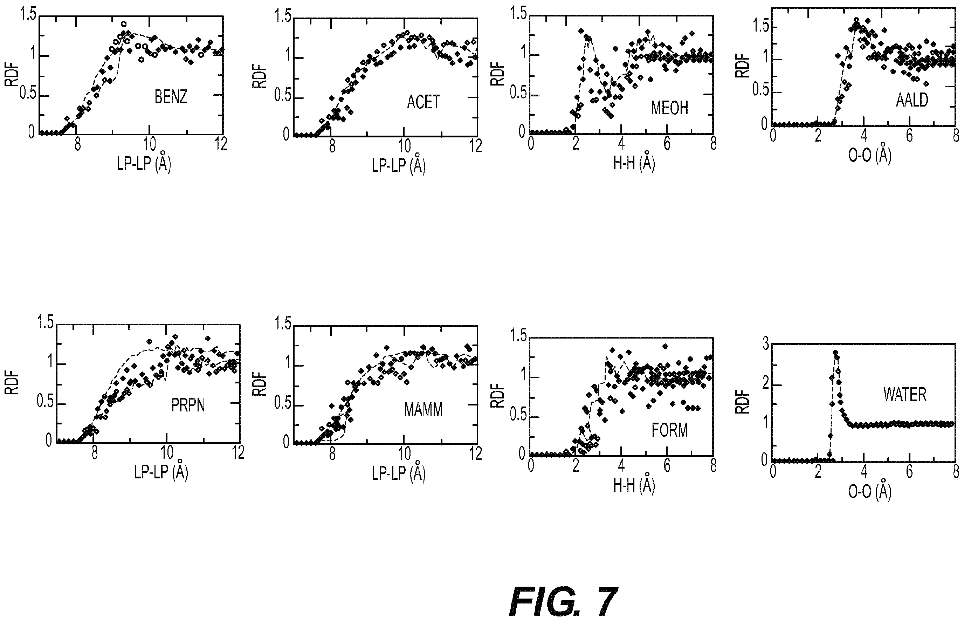

FIG. 7 depicts radial distribution functions (g(r)) for the solute atoms from Scheme I and II GCMC-MD and PBC MD simulations. Straight line: MD with PBC replicating Scheme I, closed circles: MD with PBC replicating Scheme II, Dashed line: GCMC-MD Scheme I, Open circles : GCMC-MD Scheme II. For benzene (BENZ), propane (PRPN), methylammonium (MAMM) and acetate (ACET), g(r) is measured across the massless particles (LP) that were added to their center of masses to prevent aggregation. For methanol (MEOH), formamide(FORM) and acetaldehyde (AALD), g(r) is measured between the polar hydrogens or oxygens.

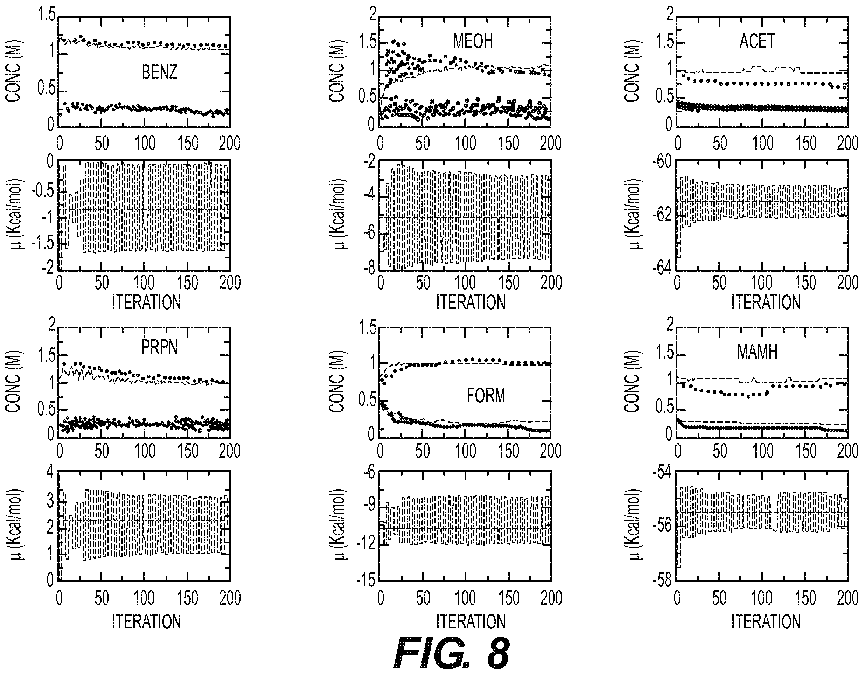

FIG. 8 shows the concentration (M) and $\mu_{ex}$ (kcal/mol), as a function of the number of GCMC-MD cycles from Scheme I and Scheme II GCMC-MD aqueous systems with $\mu_{ex}$ fixed at HFE (line and closed circles) or fluctuated by $d\mu_{ex}$ (closed squares and open circles) respectively.

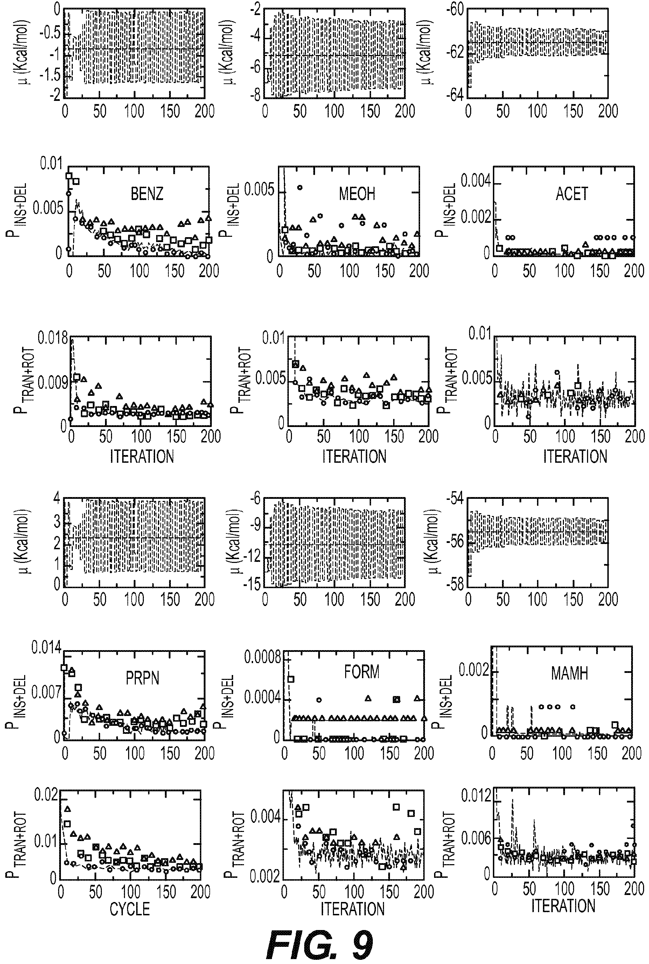

FIG. 9 depicts $\mu_{ex}$ (kcal/mol), probabilities of insertion+deletion ($P_{ins+del}$) and translation+rotation ($P_{trans+rot}$) as a function of the number of GCMC-MD cycles from Scheme I and Scheme II GCMC-MD of aqueous systems with $\mu_{ex}$ fixed at HFE (black line and closed circles) or fluctuated by $d\mu_{ex}$ (open squares and open triangles) respectively.

FIG. 10 illustrates an example setup for the Scheme I GCMC-MD with the T4-L99A mutant. The GCMC-MD is restricted to the 20 Å radius active sphere (system A) with the center at the active site of the T4-L99A mutant defined by residues Ala 99 and Met 102. The system A is encompassed in the system B which is a PBC box with walls about 12 Å away from the protein surface. System B contains both waters and benzenes at 55 M and 1 M, respectively. Waters and benzenes within the active sphere are denoted by dashes and ball-stick representations respectively.

FIG. 11 depicts the overlap coefficient (OC) between two spheres of radius R, as a function of distance of separation between them.

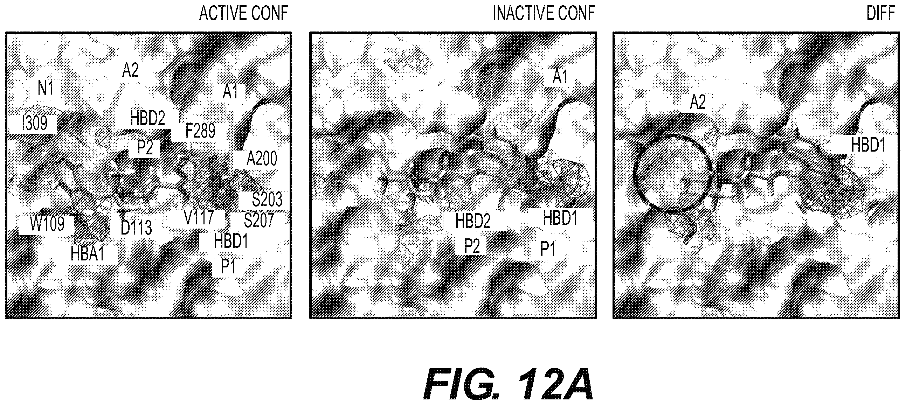

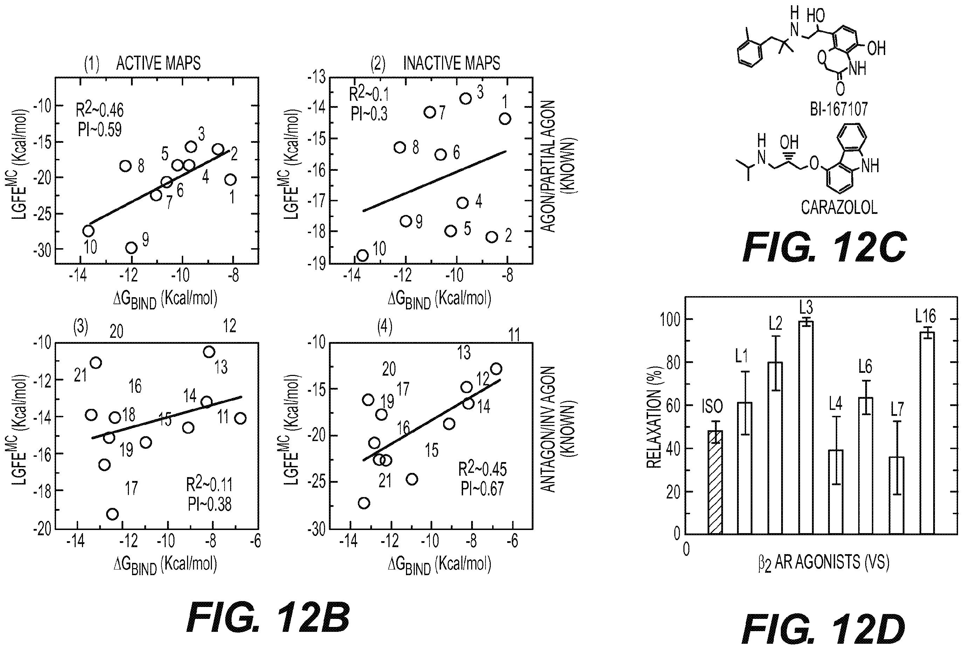

FIG. 12 depicts (A) GFE FragMaps from the B2A (left) and B2I (center) simulations overlaid on the active (PDB: 3P0G) and inactive (PDB: 2RH1) states of the $\beta_2$AR with ligands BI167107 and carazolol, respectively; receptors atoms occluding the view of binding pocket were removed. Differential maps (right) highlight differences between the two states. HBACC and HBDON FragMaps are set to a cutoff of -0.5 kcal/mol, while APOLAR, NEG and PDON FragMaps are set to a cutoff of -1.2 kcal/mol and the color for nonpolar (APOLAR), neutral donor (HBDON), neutral acceptor (HBACC), negative acceptor (NEG) and positive donor (POS) FragMaps are green, blue, red, orange and cyan, respectively; (B) LGFE scores are obtained from MC conformational ensembles of the known agonists, partial agonists (numbered 1-10), antagonists and inverse agonists (numbered 11-21) in both the B2A and B2I FragMaps. LGFEs and the experimental ΔG$_{bind}$ values correlate well for agonists and partial agonists with the B2A FragMaps (1), and the antagonists/inverse agonists with the B2I FragMaps (4);(C) Relaxation response of tracheal rings with the top 7 of the 15 selected ligands from the virtual screening (VS) studies. Isoproterenol (Iso, blue) is used as control.

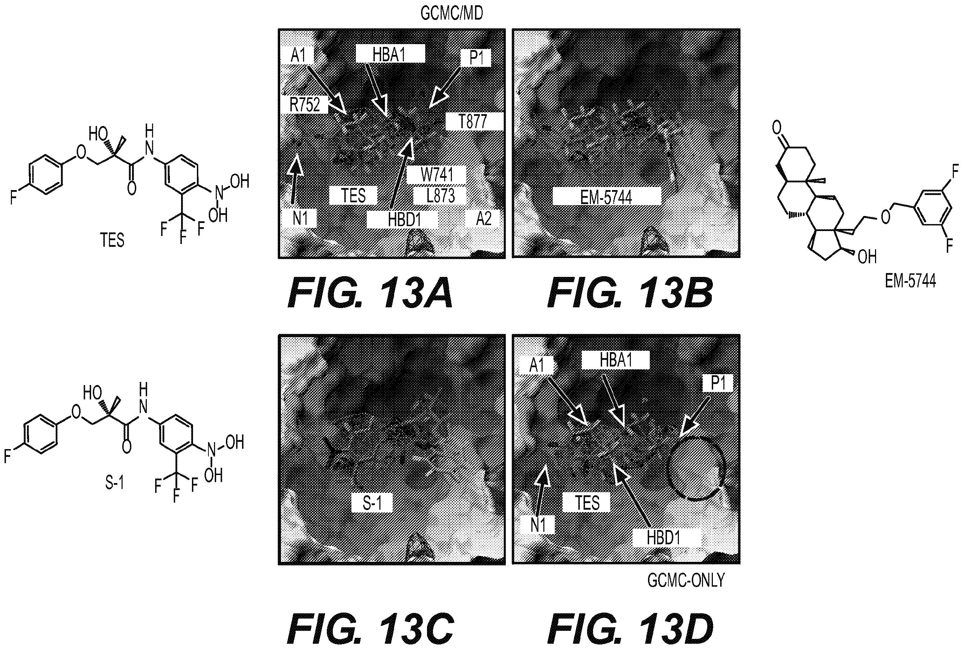

FIG. 13 depicts FragMaps overlaid on the LBP of AR (PDB 2AM9) with ligands A) TES, B) EM-5744 and C) S-1 in the crystallographic orientations. D) FragMaps from the GCMC only simulation. Receptor atoms occluding the view of the binding pocket were removed to facilitate visualization. The color for nonpolar (APOLAR), neutral donor (HBDON), neutral acceptor (HBACC), negative acceptor (NEG) and positive donor (POS) FragMaps are green, blue, red, orange and cyan, respectively. APOLAR, HBACC and HBDON FragMaps are set to a cutoff of -0.5 kcal/mol, while NEG and POS are set to -1.2 kcal/mol. Distinct FragMap affinities that overlap with the functional groups of the ligands are indicated by arrows colored the same as the FragMaps. D) The absence of protein flexibility in GCMC-only simulations leads to a general decrease in the spatial extent of the FragMaps and omission of the APOLAR FragMap A2 that is in the vicinity of the crystallographic conformations of the second phenyl rings of B) EM-5744 and C) S-1. MC sampling of ligands, EM-5744 and S-1 yields conformations (yellow) distinct from the crystal (cyan), in B and C.

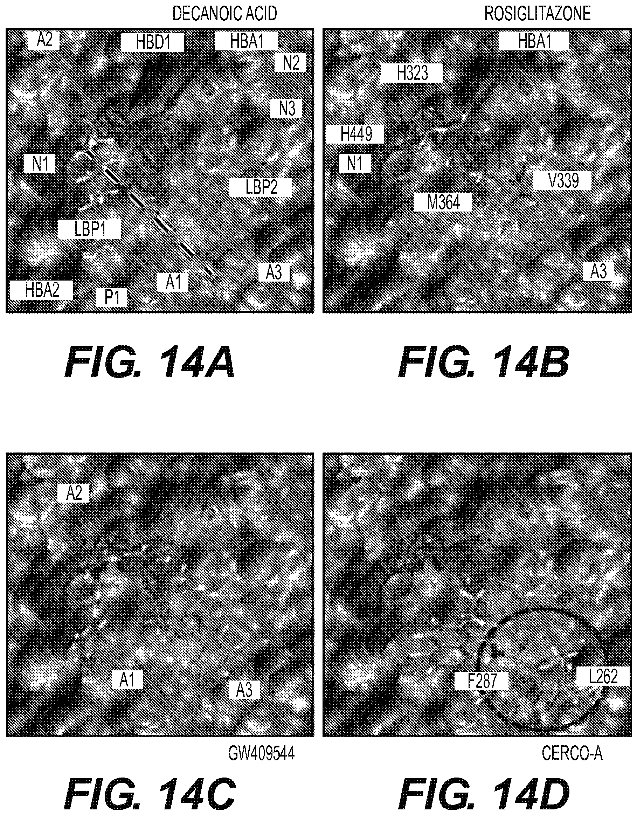

FIG. 14 depicts PPARγ FragMaps overlaid on the LBP of PPARγ (PDB 3U9Q) with ligands A) decanoic acid, B) Rosiglitazone (PDB:2PRG,) C) GW409544 (PDB: 1K74) and D) Cerco-A (PDB:3B1M) in their crystallographic orientations; receptor atoms occluding the view of the binding pocket were removed to facilitate visualization. HBACC and HBDON FragMaps are set to a cutoff of -0.5 kcal/mol, while APOLAR, NEG and PDON FragMaps are set to a cutoff of -1.2 kcal/mol. No FragMaps were found to overlap with the dibenzofurancaboxamide of Cerco-A (D).

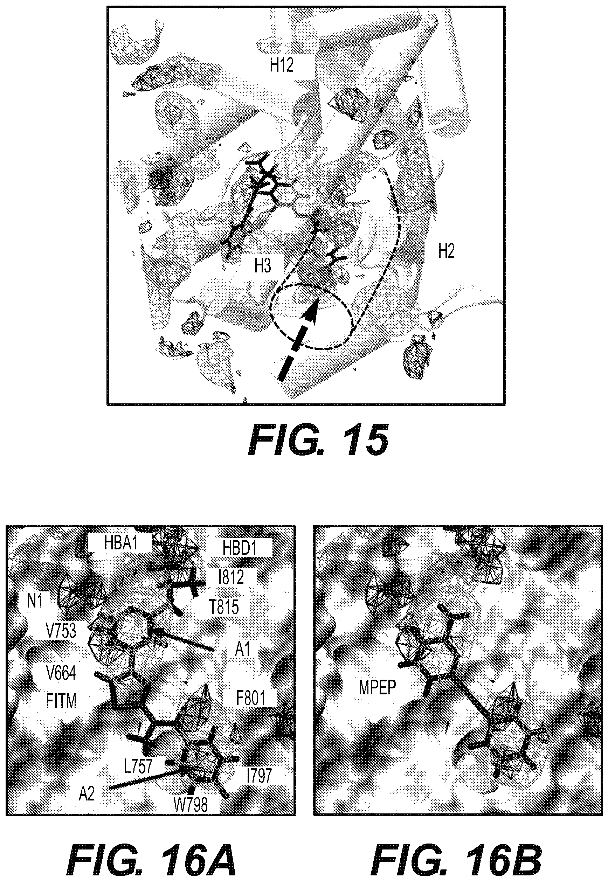

FIG 15 depicts FragMaps trace the possible passage of entry of a ligand in the PPARγ. Ligand GW40944 is shown in the LBP.

FIG. 16 depicts GFE FragMaps overlaid at the partially occluded LBP of mGluR1 with ligands A) FITM and B) MPEP. Protein atoms occluding the view of the pocket were removed to facilitate visualization. All FragMap contours are displayed at -1.2 kcal/mol and the color for nonpolar (APOLAR), neutral donor (HBDON), neutral acceptor (HBACC), negative acceptor (NEG) and positive donor (POS) FragMaps are green, blue, red, orange and cyan, respectively.

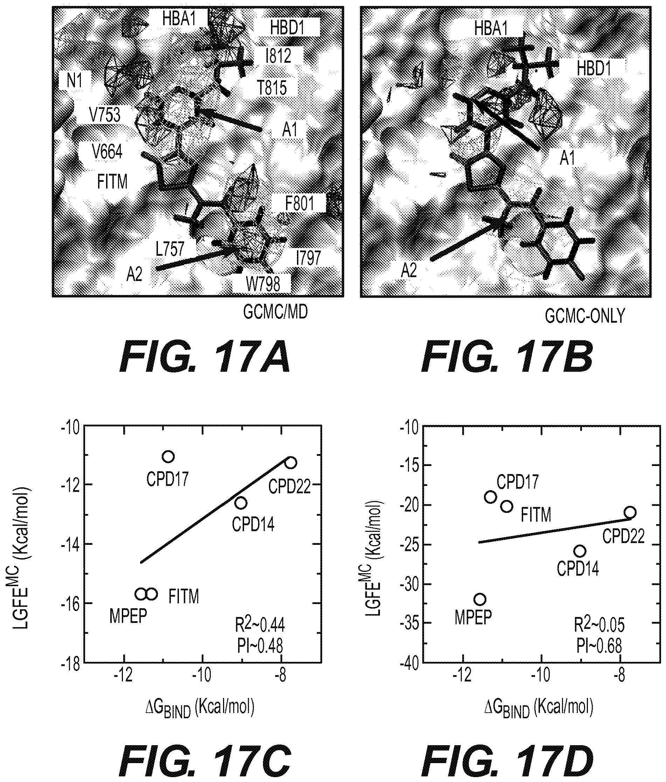

FIG. 17 compares FragMaps between A) GCMC/MD vs. B) GCMC-only simulations for the mGluR. All FragMap contours are displayed at -1.2 kcal/mol. C) LGFEs calculated using the FragMaps from GCMC/MD correlated well with $\Delta G_{bind}$ while the correlation was lost when D) the LGFEs were calculated using the FragMaps from GCMC-only simulations.

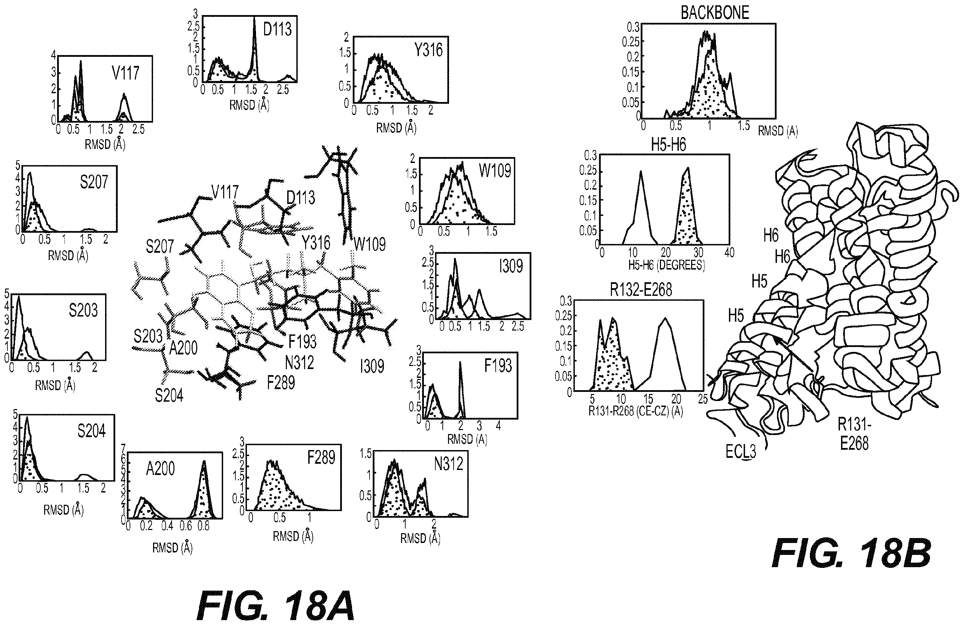

FIG. 18 depicts the Structural analysis of B2A and B2I. A) Distributions of RMSD of selected side chains identified to be pertinent for the ligand binding through the 10x50 ns GCMC/MD simulation of B2A (orange) and B2I (green). Polar residues are colored green, hydrophobic residues are colored purple and negatively charged Asp is colored red. BI-167107 and the carazolol are colored orange and green respectively. B) The inactive (green) and the active (orange) conformations of the β2AR are maintained through the simulations as evidenced by the distribution of angle between the TM helices H5 and H6 and the distance between atoms CE and CD across the salt-bridge forming residues R131-E268.

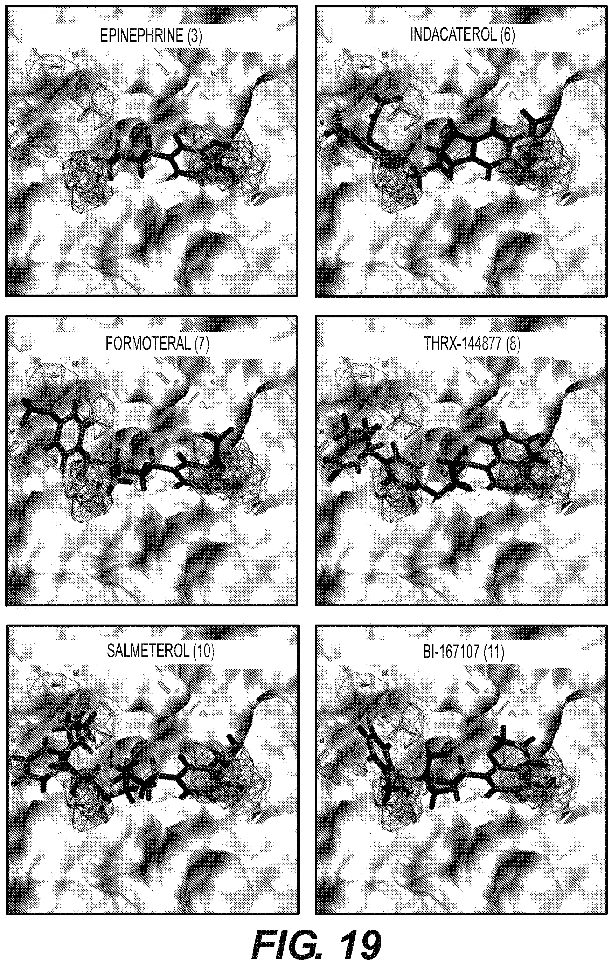

FIG. 19 depicts the binding modes of some agonist and partial agonists in the activated conformation of B2A from MC sampling of the ligands in the field of the FragMaps from the B2A SILCS-GCMC/MD.

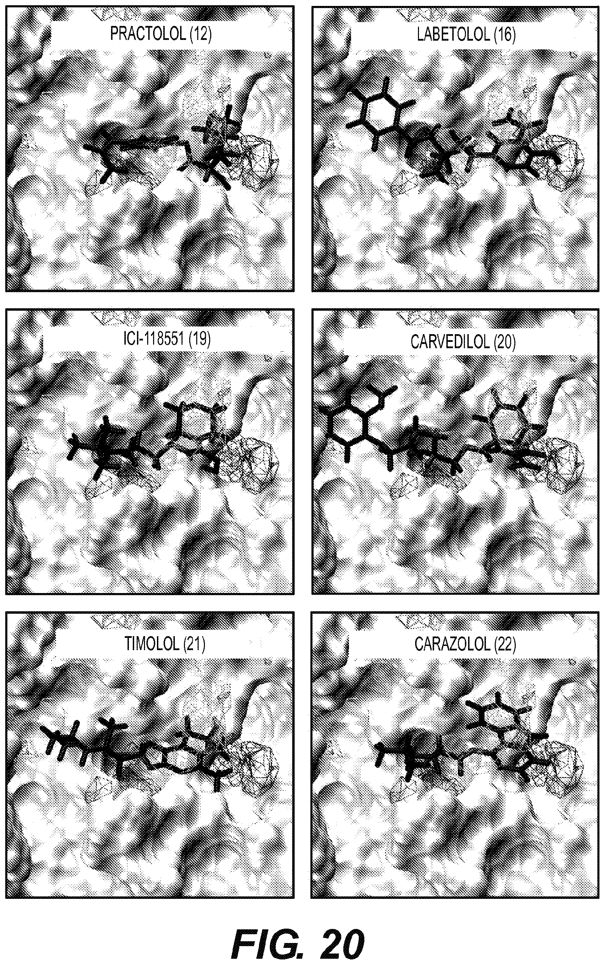

FIG. 20 depicts the binding modes of some antagonists and inverse agonists in the inactivated conformation of the β2AR from MC sampling of the ligands in the field of the FragMaps from B2I SILCS-GCMC/MD.

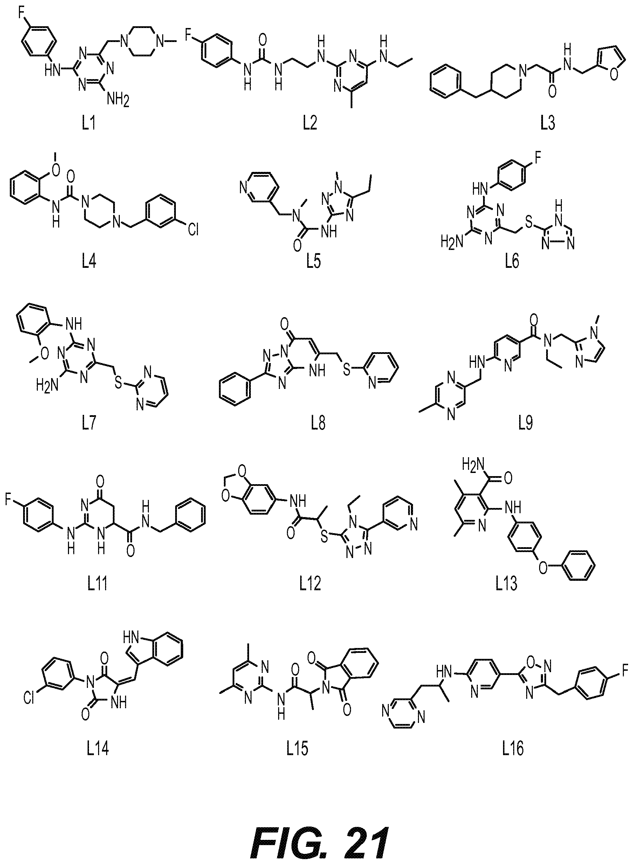

FIG. 21 depicts the structures of the fifteen ligands selected for functional assessment studies from virtual screening driven by differences in FragMaps between the active and inactive states of β2AR.

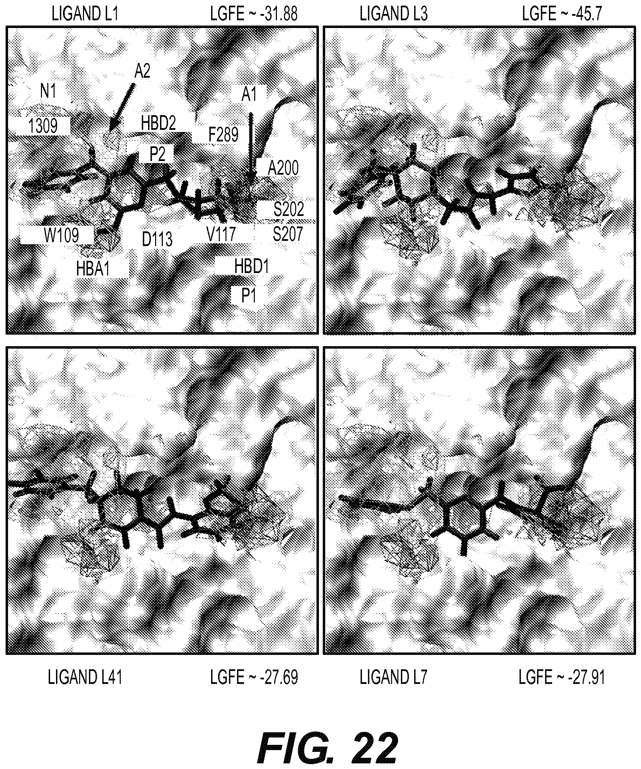

FIG. 22 depicts the docked conformations of shortlisted ligands L1, L3, L4 and L7 which overlap well with the different FragMaps from B2A simulations, leading to good LGFE scores.

DETAILED DESCRIPTION

The invention is as defined in the appended claims. Computer assisted methods are provided for using organic solutes to sample aqueous and heterogeneous environments. In the following description, for the purposes of explanation,

6

numerous specific details are set forth in order to provide a thorough
understanding of the present invention. It will be apparent, however, to one skilled in the art that the present invention may be practiced without these specific details. Still other aspects, features, and advantages of the invention are readily apparent from the following detailed description, simply by illustrating a number of particular embodiments and implementations, including the best mode contemplated for carrying out the invention. The invention is also capable of other and different embodiments, and its several details can be modified in various obvious respects, provided that they are in accordance with the appended claims. Accordingly, the drawings and description are to be regarded as illustrative in nature, and not as restrictive.

The methods of the present invention and systems in accordance therewith allow for investigations of the excess chemical potential ($\mu_{ex}$) of solutes in aqueous solution, including solutions containing multiple solutes. Central to the approach is the use of a fluctuating $\mu_{ex}$ over the GCMC portions of the simulations. This leads to convergence of $\mu_{ex}$ for given solute(s) and environment, as defined by the user, based on the target concentration and the maintenance of solute sampling once the system has converged with respect to $\mu_{ex}$ or target concentration. In addition to probing $\mu_{ex}$ required to maintain the solutes at their target concentration in aqueous environments, the iterative GCMC-MD with fluctuating $\mu_{ex}$ approach is also useful for efficient solute sampling.

It is well accepted that targeting ligands to occluded ligand binding pockets (LBP) in proteins with minimal or no accessibility to the surrounding environment is challenging. As the efficacies of ligands of both GPCRs and nuclear receptors (NRs) are known to be coupled to small conformational changes in their binding sites, accurate modeling of these sites is critical for future development of therapeutic agents for a wide range of diseases.

The SILCS methodology can be used to map the free energy affinity patterns of functional groups at protein surfaces, including LBPs. This method accounts for the conformational flexibility of proteins, chemical space of ligands, and explicit solvent by running molecular dynamics (MD) of the target protein in an aqueous solution of small solute molecules representative of different chemical functional groups. In one embodiment, the affinity patterns of these functional groups are obtained in the form of discretized probability, or, free energy maps, called FragMaps. Inclusion of protein flexibility and explicit solvent representation in the SILCS method is particularly important given the known conformational changes within the binding pocket upon ligand binding and the competition with and displacement of waters by ligands.

As further described below, the SILCS method was successful in mapping the functional group requirements of ligands for a range of macromolecules. To probe occluded LBPs, SILCS is coupled with an iterative Grand-Canonical Monte-Carlo (GCMC) and MD methodology. GCMC drives the sampling of small solutes and explicit solvent in LBPs while MD allows for conformational sampling of the macromolecules in the presence of solutes and water, which is useful in exploring cryptic pockets absent in apo crystal structures that are known to serve as binding sites.

The present invention describes the use of SILCS-GCMC/MD to map the functional group affinity patterns of the occluded pockets of the following therapeutically important NR proteins and GPCRs. Exemplary therapeutically important macromolecules are the androgen receptor (AR), the peroxisome proliferator-activated receptor-y (PPARy), nuclear receptors as well as GPCRs such as the metabotropic glutamate receptor (mGluR) and $\beta_2$-adrenergic receptor ($\beta_2$AR). The method was utilized to predict the relative binding affinities of ligands using a ligand grid free energy (LGFE) scoring scheme which, is further described below, incorporates protein conformational flexibility parameters. The method also is capable of distinguishing between active and inactive states of the $\beta_2$AR through differences in the affinity patterns of ligands across these states. From a drug discovery perspective, such information is useful for distinguishing the function of ligands. Validating this capability is the ability to utilize FragMaps differences to identify new agonists of $\beta_2$AR, compounds that have the potential to be developed into therapeutic agents for the treatment of asthma and other obstructive pulmonary diseases.

In one embodiment, the method may be used to sample the configurational space of an occluded pocket in a macromolecule. This sampling may be performed on a single solute in conjunction with water wherein the concentration of the solute *in silico* is in a range from about 0.01M to 1000M so as to estimate the binding affinity of the solute or a complex mixture of solutes for the occluded pocket of a macromolecule and to map the functional preference of groups for the occluded (binding) pocket of the macromolecule. In certain examples, the solute concentration is 1M because it corresponds to concentration at the standard state, which provides the experimental free energy of binding. When this is analyzed in the context of the SILCS methodology, the approach is shown to qualitatively reproduce the binding orientation of known ligands as well as quantitatively rank the order of the binding affinities of ligands. Such information concerning the functionality and binding orientation of ligands assists with computer aided drug design methods that are targeted towards the identification of high affinity ligands for macromolecules with deep or occluded binding pockets such as protein nuclear factors and GPCRs.

The present invention includes, therefore, methods for solute sampling in explicit solvent aqueous systems and solvated protein environments using iterative GCMC simulations. The method of the present invention is a computational method as defined in claim 1.

In certain embodiments, the methods of the present invention may further generate an output of the spatial distributions of the one or more solutes and water. Among other things, the output may be used to assist with computer-aided drug design.

Details of the spatial distribution will depend on the number of repetitions of steps 2) and 3) with the user defining when a satisfactory spatial distribution is obtained. Typically, a user stops repeating steps 2) and 3) when the details of the spatial distribution of the one or more solutes and water no longer undergo a significant change with each additional repetition as defined by the user. This scenario may be referred to as a converged spatial distribution. In an aqueous solution, for example, a converged spatial distribution will be homogeneous (i.e. the same everywhere for water and all solutes). Alternatively, when the system is heterogeneous (i.e. contains a macromolecule as defined by the appended claims) the spatial distribution of solutes and water will also be heterogeneous. However, the spatial distribution can be defined as converged if it does not undergo significant changes with more repetitions, where significant is defined by the user.

In the context of the present invention, the oscillating $\mu_{ex}$ permits the determination of the spatial distribution of the solutes and water and the term "convergence" refers to the number of GCMC steps at which the spatial distribution of water and solutes no longer changes. While $\mu_{ex}$ can still oscillate for each sampling of the spatial distribution, the average value of $\mu_{ex}$, however, cannot change once convergence is obtained. In one embodiment convergence of spatial distributions is identified using overlap coefficients. In one embodiment spatial distributions are considered converged when the overlap coefficient is greater than 0.4. In an alternate embodiment, convergence is identified when the deviation of the concentration of a solute in the system undergoing sampling of the spatial distribution is consistently less than 10% from the target concentration for at least the last 20 sampling cycles.

The system may include only water, one or more solutes, or a combination of water and the one or more solutes. The target concentration may be set to any desired level. However, in one particular embodiment having only water and one solute, the target concentration of the solute is set to 1M and the target concentration of the water is set to 55 M.

The number of GCMC operations in step 2) may be greater than one for each cycle. In certain embodiments GCMC operation is performed from about 100 to 100,000 times. However, less than 100 or more than 100,000 GCMC operations can be performed if necessary. The GCMC operations may be divided in any fashion amongst the one or more solutes and water. In certain embodiments, the GCMC operations are divided equally between the one or more solutes and water. The proportion of GCMC operations for each of the one or more solutes may also be assigned in any ratio desired. However, in certain embodiments, the proportion of GCMC operations is assigned based on the target concentration of each of the solutes and water. In certain embodiments, the $\mu_{ex}$ of the one or more solutes and water is alternately increased and decreased during the GCMC operations across consecutive cycles involving steps 2) and 3). The alternating process of increasing or decreasing the $\mu_{ex}$ of the one or more solutes and water is repeated until the overall spatial distribution no longer changes or reaches an acceptable level of change.

Macromolecules are also present in the systems in accordance with the present invention. They are selected from a protein, RNA, DNA, carbohydrate, lipid, organic chemical, inorganic chemical, or any combination thereof. Said macromolecules are generally greater than 1000 daltons, and less than 10,000,000 daltons. Generally, the macromolecules are in a range of 5,000 to 500,000 daltons. In certain systems that comprise a macromolecule, the sampling method of the present invention further includes a step wherein $\mu_{ex}$ of the one or more solutes and water is alternately increased and decreased during the GCMC operations across consecutive cycles involving steps 2) through 3) after the concentration of the one or more solutes and water reach their target values. In embodiments that comprise one or more macromolecules, the spatial distribution of the one or more solutes may be used to identify preferential affinity of each of the solutes for the macromolecule. This can be achieved by determining the relative probability that the one or more solutes are spatially located adjacent or in one of the macromolecules versus the other or to the remainder of the system. The same holds for multiple sites on a single macromolecule. The binding affinity can be estimated based on the probability of the solutes being in a site relative to the remainder of the system or relative to another site on the macromolecule itself or versus the other macromolecule.

In yet other embodiments, the spatial distribution following step 2) is sampled using molecular dynamics (MD) simulations. A MD simulation after the GCMC allows for both conformational sampling of the solutes and configurational sampling of the aqueous system and the macromolecule. To achieve satisfactory convergence as defined by the user, the process of GCMC-MD is repeated through multiple iterations, with the $\mu_{ex}$ of the species being studied systematically oscillated over the iterations to drive the solute and water exchanges. Many of the examples provided herein are described using GCMC-MD but MD simulation may not be used in all embodiments of the present invention.

In certain embodiments, the system used in the methods described herein is a finite spherical system. The difference between the radii of the systems may be 0.1 Å to 1000Å. In certain embodiments, the difference between the radii is or is approximately 5Å. While specific examples provided herein are taught with a spherical system, any defined region of space may be used.

The theory of the GCMC is described below. There are four possible GCMC moves on a molecule, M (i.e., solute or water): Insertion: brings M into the system A from the reservoir; deletion: removes M from the system A and moves it back into the reservoir; translation and rotation: M is translated/rotated within a sub-volume surrounding the original location of M in system A. The probabilities of these moves as governed by the Metropolis criteria are:

$$P_{insert} = \min\left\{1, \frac{f_n}{n+1} e^{B - \beta \Delta E}\right\}$$

$$P_{delete} = \min\left\{1, \frac{n}{f_{n-1}} e^{-B - \beta \Delta E}\right\}$$

$$P_{trans/rot} = \min\left\{1, e^{-\beta \Delta E}\right\}$$

$$\text{where } B = \beta \mu_{ex} + \ln \overline{n} \text{ and } \overline{n} = \overline{\rho v} \qquad (1)$$

$\mu_{ex}$ is the excess chemical potential, $\overline{n}$ is the expected number of molecules, $\overline{p}$ is the density, $\overline{v}$ is the volume of system A, $f_n$ is the fractional volume of the subspace where the insertion attempts are made, $\Delta E$ is the change in energy due to a move, $\beta$ is $1/k_B T$, $k_B$ is the Boltzmann constant and T is temperature (300 K in the present study). Through the GCMC simulation, the volume of the simulation system A, the total energy and the total number of particles between the system A and its reservoir are typically fixed. However, in certain embodiments the total energy and the total number of particles between the system A and its reservoir can vary.

As seen in Eqn.1, the expected number of molecules of each solute "$\overline{n}$" is calculated from the target concentration of each solute ($N_{tgt}$), and the volume of the system A. Thus, the interactions of each solute with the other molecules in the simulation system A ($\Delta E$) and the supplied $\mu_{ex}$ determines the solute populations of GCMC simulations. Since, the move probabilities of the individual solutes or waters are driven by their $\overline{n}$ and $\mu_{ex}$ values, when the $\mu_{ex}$ of a solute is less than the work needed to move a molecule from the gas-phase reservoir to the system A, a decrease in the concentration of the solute from the system A will occur. Likewise, when the $\mu_{ex}$ supplied is more than the needed work, an increase in solute concentration will occur in system A. Consequently, the value of $\mu_{ex}$ supplied to the GCMC simulation may be varied based on the number of particles in the simulation system and the expected number of molecules of each solute $\overline{n}$.

With $\overline{n}$ used as a target for each solute for the GCMC calculation, the simulation system A is defined to contain water at bulk-phase concentrations of 55 M. Through the iterative GCMC-MD simulations, the number of solutes and the water in system A vs. their expected number ($\overline{n}$) is used as a guide to vary their respective $\mu_{ex}$ through each subsequent GCMC iteration. The simulations start with the value of $\mu_{ex}$ for solutes and water set to 0 in the first iteration. Over every subsequent iteration or subset of iterations, the value of $\mu_{ex}$ for solutes and water is varied by a magnitude that is governed by the deviation of the solutes and water in system A from their respective target number $\overline{n}$. As the concentrations of the solutes and the waters reach their target number, the width of the variation of each $\mu_{ex}$ is decreased and this defines the onset of convergence. Thus, in the standard state simulations with only one type of solute at a concentration of 1M in water, the hydration free energy (HFE) can be calculated from the value of $\mu_{ex}$ at convergence. The oscillation of $\mu_{ex}$ values for water and solutes is continued following convergence to facilitate the spatial sampling of solutes under equilibrium conditions.

Scheme 1 of Figure 1, illustrates a standard state simulation in which system A comprises a single type of solute at a concentration of 1 M. Scheme II illustrated in Figure 1, however represents a simulation in which the system comprises an aqueous mixture of multiple solutes . As illustrated in Figure 1, System A, is a spherical region of radius, $r_A$, in which GCMC moves are performed. As further illustrated in this figure, separate reservoirs of solutes and water are coupled to system A. System A also is immersed in a larger system B, which includes additional water. For the bulk aqueous systems, system B is a larger sphere of radius $r_B = r_A + dr$. In one embodiment dr=5 Å. The larger system B limits edge effects, such as those arising from hydrophobic solutes occupying the edge of the system A (Fig. 6). Alternatively, system B may be treated using periodic boundary conditions (PBC) and/or include other chemical entities in addition to water. Figure 10 illustrates an exemplary T4-L99A system for GCMC-MD simulations. In this figure a periodic system B is immersed in a spherical system A.

The iterative GCMC-MD procedure is performed as follows:

1) Run i steps of GCMC to exchange solutes and waters between their respective reservoirs and the simulation system A. The i steps were divided between each of the M solutes and water. In the present study $\dfrac{i}{2}$ and $\dfrac{i}{M+1}$

GCMC moves (insertion/deletion/translation/rotation) were attempted for the solutes ($F_1$, $F_1$, $F_2$,...$F_M$) and the water, in Scheme I and Scheme II, respectively. However, there is no requirement that the number of GCMC moves for the different solutes and water, have to be equal. The order in which the four possible GCMC moves are attempted, and the molecule (solutes or water) on which the move is performed is randomized. In the first iteration, the value of $\mu_{ex}$ for solutes and water was set to 0. The radii of the water and the solute sphere(s) subjected to GCMC moves were set to $r_A$ through the GCMC process, though the energetic interactions associated with the moves also includes contributions from any water molecules, or other chemical entities, outside of the GCMC sphere that is defined as system B.

2) After the GCMC, $j$ steps of MD were run on combined systems A and B. For the finite spherical systems the solutes were retained within the spherical dimensions of system A, ($r_A$) using several known methods. In one exemplary embodiment this was performed through the use of harmonic flat-bottom spherical restraints as described by Caleman C, et al., (2011), Proc Natl Acad Sci USA 108(17):6838-6842. Additionally, water and any other molecules in system B encompassing system A were subjected to i) harmonic flat-bottom spherical restraints with $r_B = r_A + dr$ when system B is spherical or ii) periodic boundary conditions.

3) In step 3, the value of $\mu_{ex}$ for solute and water, ($\mu_P$ (P=$F_1$, $F_2$,..., $F_M$ solutes or W, water) was varied by $d\mu_P$. This new value of $\mu_P$ was used in the next iteration of GCMC. The magnitude of $d\mu_P$ was determined by the deviation of the current number of solutes/water, $N_P$ (P=$F_1$, $F_2$,..., $F_M$, or W) in system A from the expected number derived from the target concentration of solutes/water ($N_P^{target}$) derived from the expected number of solutes/water.

$$\mu_P = \mu_P + d\mu_P$$
where
$$d\mu_P = d\mu_P \times 5, \text{ when } N_P = 0; \text{ for cycle } 1, |d\mu_P| = 0.5$$
else
$$d\mu_P = \begin{cases} d\mu_P \times \dfrac{N_P^{target}}{N_P}, & N_P < 0.7 N_P^{target} \\ \text{rand}\left((d\mu_P - 0.5), (d\mu_P + 0.5)\right), & 0.7 N_P^{Target} < N_P < 2 N_P^{target} \\ -d\mu_P \times \dfrac{N_P}{N_P^{target}}, & N_P > 2 N_P^{target} \end{cases}$$

$$(2)$$

With the variation of $d\mu_P$ system-dependent, various schemes (standard state, aqueous mixture, heterogeneous systems) can be generated using Eqn. 2. It should be noted that different schemes can be applied to vary $d\mu_P$.

4) The new value of $\mu_{ex}$ from step 3 of the current iteration was then used to perform $k$ iterations (steps 1-3), of a GCMC-MD simulation.

The GCMC simulations were run using an in-house C++ code that implemented the grid-based GCMC scheme with the cavity-bias algorithm to drive solute and water exchanges between their reservoirs and the aqueous system A. Solute empirical force-field parameters were obtained from CGenFF the general force field in CHARMM and TIP3P, a model for water used in GCMC and MD simulations. The solute molecules chosen for the present study represent different chemical functionalities including apolar molecules such as benzene and propane, neutral polar molecules such as acetaldehyde, methanol and formamide, and the negative and positively charged molecules such as acetate and methylammonium, respectively.

To prevent aggregation of hydrophobic and charged solutes, and promote faster convergence, a repulsive energy term was introduced only between benzene:benzene, benzene:propane, propane:propane, acetate:acetate, acetate:methylammonium and methylammonium:methylammonium molecular pairs. This was achieved by adding a massless particle to the center of mass of benzene and the central carbon of propane, acetate and methylammonium. Each such particle does not interact with any other atoms in the system but only with other particles on the hydrophobic or charged molecules through the Lennard-Jones (LJ) force field term using the following parameters: $\varepsilon = 0.01$ kcal/mol; and $R_{min} = 12.0$ Å. All LJ force field terms and coulomb interactions were calculated during GCMC (i.e. there was no truncation of non-bonded interactions), including the interactions with system B. Simulations are initiated with an empty system A. The randomized GCMC process with the solutes and the waters was initially run in multiples of 50,000 moves until the water molecules in the system reach a bulk concentration of 55 M. At each point in this iterative process of 50,000 GCMC moves, the $\mu_{ex}$ of the solutes and water was increased by 1 kcal/mol to accelerate the water and solute accumulation in system A. During this process, the concentration of the solutes may increase beyond their target values. After the bulk water concentration of 55M is attained, the GCMC-MD procedure as described above in steps 1-4 was run after resetting $\mu_{ex}$ of the respective fragments and the water to 0. Over 50 iterations of the GCMC-MD in which the $\mu_{ex}$ values were varied, the concentrations of solutes approach their respective target values. For each iteration, 50,000 and 100,000 GCMC moves were run for Scheme I and Scheme II, respectively.

The GROMACS package (version 5.0) was used for all MD simulations. For the aqueous systems, for each iteration, the combined systems A and B, including all the solutes and the waters were simulated for 500 ps using a leap-frog integrator (GROMACS integrator "md"), having a 2 fs time-step, at 300 K through a Nose-Hoover thermostat. The LINCS algorithm was used to constrain water geometries and all covalent bonds involving a hydrogen atom, while van der Waals (vdW) and electrostatic interactions were switched off smoothly over a range of 8-10 Å. Solutes and water molecules were held within the spherical dimensions of systems A or B by applying harmonic flat-bottom restraints with a force constant of 1.2 kcal/mol/Å$^2$ on the following solute or water atoms: the massless particle at the geometric center of benzene, propane, acetate and methylammonium, the carbon atoms of the acetaldehyde, methanol and formamide and the oxygen atom of water. For the bulk aqueous systems, the radius, $r_B$ of the harmonic flat-bottom restraints used to define system B applied only to water and was 5 Å larger than the radius $r_A = 20$ Å defining the restraint applied to the solutes in system A.

For the T4-L99A system, PDB coordinates 181L with the benzene ligand was used following deletion of the ligand. Briefly, the T4-L99A structure was inserted into boxes replicating Scheme I and Scheme II systems containing 1 M of benzene and 0.25 M each of the different fragments (benzene, propane, acetaldehyde, methanol, formamide, acetate and methylammonium), respectively. Akin to the aqueous systems, 10 such boxes were built for Scheme I with 1M benzene and Scheme II with the multiple solutes, with the solutes randomly inserted in each of the boxes. These systems were minimized over 1000 iterative steps using the steepest descent algorithm in the presence of periodic boundary conditions (PBC). The systems were then equilibrated for 250 ps by periodic reassignment of velocities. The leap frog version of the Verlet integrator with a time step of 2 fs was used for heating and equilibration. Long range electrostatic interactions were handled using the particle-mesh Ewald method with a real space cutoff of 12 Å. A switching function was applied to Lennard-Jones interactions at 12 Å, and a long-range isotropic correction was applied to the pressure component for Lennard-Jones interactions beyond the 12 Å cutoff length. During minimization and equilibration, harmonic positional restraints with a force constant of 2.4 kcal/mol /Å$^2$ were applied to protein non-hydrogen atoms. For MD simulations in the iterative protocol, the position restraints were removed and replaced by weak restraints applied only to the backbone C-alpha carbon atoms of the protein with a force constant ($k$ in $1/2 k\delta x^2$) of 0.12 kcal/mol /Å$^2$. This was done to prevent the rotation of the protein in the simulation box and to prevent the potential denaturation of the protein due to the presence of a highly concentrated fragment solution (17).

The GCMC-MD iterations are repeated until convergence is obtained as defined by the user. Typically, about 200 GCMC-MD iterations are needed for the aqueous systems, yielding 100 ns of MD simulations and 10 and 20 million MC steps for Scheme I and Scheme II, respectively. Furthermore, to ensure sufficient sampling and convergence, 10 separate GCMC-MD simulations were run for Scheme II and for each solute in Scheme I resulting in a cumulative 1 $\mu$s ($10 \times 100$ ns) of MD for Scheme II and each solute in Scheme I. Runs differ through a randomly generated seed for both GCMC and the leap-frog MD integrator operations in each iteration. GCMC-MD of the protein systems are repeated over 100 iterations, yielding a cumulative 500 ns of MD over the 10 separate simulations from both Scheme I and Scheme II systems. In the context of Scheme I, the GCMC-MD simulations would allow calculation of the HFE corresponding to a 1 M standard state aqueous system and the binding affinity of solutes to the protein. With the solute mixture in Scheme II the approach would permit determining the $\mu_{ex}$ required to drive sampling of the distribution of solute molecules in a heterogeneous aqueous system and determine solute affinity patterns around the protein site.

In the initial calculations, when solute and solvent in the finite spherical systems shared the same spherical boundary, the nonpolar molecules only sampled the surface of the spherical system (FIG. 6). This is due to the favorable interactions of the nonpolar solutes with the nonpolar, vacuum environment outside of the spherical simulation system. To overcome this, the radius of the simulation system for water was increased by 5 Å, such that the "pancaking" of nonpolar molecules is avoided as they stay fully hydrated. The use of such an extended system can alleviate other potential edge effects that other solutes may encounter. In one aspect of the invention, the larger system B can extend to any distance beyond the solute restraints defining system A. The larger system B also can be modeled using the periodic boundary conditions (PBC), that are described in the calculations for the T4-L99A system.

Two types of aqueous systems were considered: 1) a system containing only one type of solute in water at a concentration of 1 M, thereby replicating the standard state of the solute and 2) a dilute aqueous mixture containing many types of solutes each at a concentration of 0.25 M. Results using both Schemes I and II are presented for aqueous systems alone, followed by a description for Scheme I and Scheme II calculations on a system that contains T4-L99A lyzozyme. This lysozyme mutant was selected as a model system as it has been widely used in experimental and computational studies of ligand binding as well as in studies evaluating the impact of mutations on protein structure and stability. The L99A mutation in the C-terminal domain T4 creates a completely buried, hydrophobic cavity of $\sim 150$ Å$^3$

that, although inaccessible in static structures, binds small hydrophobic ligands in a rapid and reversible manner. Such an occluded pocket offers a rigorous test of the sampling effectiveness of the presented GCMC-MD methodology, including a quantitative evaluation of the approach.

Briefly, the GCMC-MD methodology can be explained as follows. GCMC was run on water and each of the different solutes in the system. Variations in the value of $\mu_{ex}$ for water and solutes for the GCMC-MD iterations was used to improve the solute exchange probabilities and simultaneously permit the determination of $\mu_{ex}$ required to maintain a defined concentration of the solutes and water molecules in the aqueous systems. This approach therefore, allows the HFE of solutes to be determined using their $\mu_{ex}$ values when the target concentration of solute in the aqueous systems is 1 M. In the presence of the protein, the GCMC-MD strategy was used to determine the binding affinities of solutes to the protein as well as to efficiently sample the spatial distribution of the solutes in and around the protein. The site identification by ligand competitive saturation (SILCS) methodology is a fragment based sampling method that maps free energy affinity patterns of functional groups at protein surfaces, including occluded ligand binding pockets of proteins. The SILCS method, therefore, permits determination of the affinity patterns of multiple solutes to the protein and can be used for rational drug design.

**Methods**

**I. Simulation Details for Scheme I and Scheme II Aqueous Systems- Molecular Dynamics with Periodic Boundary Conditions**

Empirical force field parameters for proteins are CHARMM36, ligand molecules were treated using CGenFF and water was treated using the TIP3P model. Simulations were performed using the CHARMM molecular simulation program. To replicate Scheme I, the number of molecules required to attain a 1M concentration of a specific solute were randomly placed in a cubic box whose sides were 50 Å each. Similarly, to replicate Scheme II, the number of molecules required to attain 0.25 M concentration of each solute type, were randomly placed in a bulk water cubic box whose sides were 50 Å each. The aqueous mixtures were minimized using the steepest descent algorithm over 5000 steps and the further minimized over another 5000 steps using the conjugate gradient algorithm in the presence of periodic boundary conditions. The systems were then heated to 300K at the rate of 5K/ps by periodic reassignment of velocities. Following this, the system was equilibrated for 200 ps using velocity reassignment. The leap frog version of the Verlet integrator with a time step of 1fs was used for heating and equilibration. Water geometries and covalent bonds involving hydrogen atoms were constrained using the SHAKE algorithm. Long range electrostatic interactions were handled with the particle-mesh Ewald method with a real space cutoff of 12 Å. A switching function was applied to the Lennard-Jones interactions at 10 Å, and a long-range isotropic correction was applied to the pressure parameter for Lennard-Jones interactions beyond the 12 Å cutoff length. Following equilibration, the aqueous mixture boxes were simulated for 15 ns with a time step of 2fs, at 300K and at 1 atm pressure using a Nose-Hoover thermostat, and the Langevin piston barostat.

**II. Generation of FragMaps**

3D probability distributions for selected atoms of solutes from the GCMC/MD and GCMC-only simulations are called "FragMaps." For the present invention, FragMaps were constructed for benzene carbons, propane carbons, methanol polar hydrogen, methanol oxygen, formamide polar hydrogens, formamide oxygen, imidazole unprotonated nitrogen, imidazole hydrogen, acetaldehyde oxygen, methylammonium polar hydrogens, and acetate oxygens. Briefly, solute atoms from 10 ps snapshots of the Scheme II aqueous mixture systems in the absence and the presence of the T4-L99A mutant GCMC-MD simulation were binned into 1 Å x 1 Å x 1 Å cubic volume elements (voxels) of a grid spanning the entire system, and the voxel occupancy for each FragMap atom type was calculated.

For the GCMC-only simulations, voxel occupancies for each FragMap atom type were obtained every 1000 steps of GCMC in every cycle. These were then normalized by the voxel occupancies of the fragments in a bulk-phase system devoid of the protein. Bulk-phase occupancies were equal across the GCMC/MD and GCMC-only simulations. The voxel occupancies of the eleven atom types were merged to create the following five generic FragMap types: (1) generic nonpolar, APOLAR (benzene and propane carbons); (2) generic neutral hydrogen bond donor, HBDON (methanol, formamide and imidazole polar hydrogens); (3) generic neutral hydrogen bond acceptor, HBACC (methanol, formamide, and acetaldehyde oxygen and imidazole unprotonated nitrogen) (4) positive donor, POS (methylammonium polar hydrogens); and (5) negative acceptor, NEG (acetate oxygens).

The probability distributions for each atom type in the absence of protein were normalized and then converted to free energies via a Boltzmann-based transform of the normalized probability to yield a grid free energy (GFE) for each fragment type "f' for the coordinates x,y,z, referred to as GFE FragMaps. All GFE values were capped at 3 kcal/mol. FragMaps were visualized as iso-contour surfaces at a GFE value of -1.2 kcal/mol, unless otherwise noted.

**III. Ligand Grid Free Energy Scoring (LGFE)**

LGFE quantifies the overlap of atoms in ligands in the ligand binding pocket (LBP) with the corresponding GFE FragMaps. Ligand atoms were classified into FragMap types, according to an assignment map. Briefly, each classified atom of a ligand with coordinates $(x_i, y_i, z_i)$ was assigned a score equal to the GFE value of the corresponding FragMap type (f), $GFE^f_{xi,yi,zi}$, of the voxel it occupies. LGFE is then the sum of each of these GFE values for all the classified ligand atoms. LGFE was calculated as Boltzmann weighted average over an ensemble of conformations obtained by MC sampling of the ligand in LBP, in the field of FragMaps.

**IV. MD conformational ensemble (for LGFE$^{MD}$):**

Initial ligand conformations were extracted from the corresponding co-crystal coordinate PDB files and the automated CGenFF parametrization algorithm was used to obtain the topology and parameters in the context of CGenFF. The ligand conformation from the co-crystal structure was extracted and aligned with the protein conformation used in the SILCS simulations. The alignment was done based on optimal alignment of the backbone $C_\alpha$ atoms in the two protein structures. The complex was minimized using the SD algorithm for 50 steps. This minimized conformation was subject to a 10 ps MD simulation, with snapshots output every 0.2 ps. During the dynamics, all protein atoms farther than 8 Å around any ligand atom were restrained using a force constant of 1 kcal/mol/Å$^2$. This process was repeated on 40 protein conformations obtained from the GCMC-MD SILCS simulations, equally spaced in time (cycle 10, 20, 30,...), yielding at total of 1000 ligands conformations for estimation of the Boltzmann weighted LGFE.

**MC conformational ensemble (for LGFE$^{MC}$):**

Sampling of the ligand was also performed in the "field" of the GFE FragMaps using Metropolis Monte Carlo (MC) steps. An in-house suite of programs was used to setup and run the MC simulations. The ligand had rotational, translational and intra-molecular degrees of freedom. The ligand had no rotational restraints, but its center of mass (CoM) was restrained to lie within 2.5 Å of the CoM of the ligand crystal conformation using a flat bottom restraint. Intramolecular degrees of freedom consisted of rotatable bonds, which were automatically detected based on the CGenFF molecular topology. All acyclic non-terminal bonds were considered rotatable, with the exception of bonds ending in methyl or $NH_3^+$ groups. The force-field terms corresponding to the intra-molecular degrees of freedom comprised of dihedral, van der Waals (vdW) and electrostatic terms. Due to the absence of protein and solvent during these simulations a distance dependent dielectric (=4|r|) was used to evaluate the intramolecular electrostatic contributions to prevent their overestimation. The energy computed during the Metropolis MC can be written as follows.

$$E = E_{vdw, intra} + E_{elec, intra} + E_{dihe, intra} + LGFE$$

For each ligand, 20 different MC simulations (each run for 10,000,000 steps; snapshots recorded every 10,000 steps) were run, where for each of them the ligand is initially randomly placed close to the protein active site. An average LGFE is first calculated over each of these 20 MC simulations. LGFE$^{MC}$ is then the Boltzmann weighted average over these LGFE values obtained across the 20 MC simulations.

**V. Determining Occludedness of the Ligand Binding Pocket**

The extent by which the binding sites are occluded is referred to as occludedness, and is calculated as the ratio of the solvent accessible surface area (SASA) of the ligands alone to the SASA when bound to the protein using ligand-protein complex X-ray structures. As shown in Table 1, these ratios vary from 0, for testosterone bound to AR up to 0.13 for both Rosiglitazone bound to PPARγ and Carazalol bound to β$_2$AR. This data suggests a high level of inaccessibility of LBPs for these therapeutically important drug targets.

**Table 1**

| Receptor | Ligand | Bulk SASA | LBP SASA | Ratio |
|----------|--------|-----------|----------|-------|
| AR | Testosterone | 402.1 | 0 | 0 |
| PPARγ | Rosiglitazone | 506.8 | 68.6 | 0.13 |

(continued)

| Receptor | Ligand | Bulk SASA | LBP SASA | Ratio |
|---|---|---|---|---|
| | Decanoic acid | 341.1 | 28.8 | 0.08 |
| mGluR | FITM | 576.8 | 43.8 | 0.07 |
| $\beta_2$AR | BI-167107 | 534.8 | 36.32 | 0.07 |
| | Carazalol | 478.6 | 62.8 | 0.13 |

## VI. GCMC-MD for Mapping Functional Group Free Energy Patterns at Occluded LBP

During GCMC, fragments and water are exchanged between their gas-phase reservoirs and a cubic region of radius 20 Å (25 Å for the PPARγ and AR) encompassing the ligand binding pocket of the protein. The excess chemical potential ($\mu_{ex}$) supplied to drive fragment exchanges is periodically fluctuated, for example, over every 3 cycles, such that the average $\mu_{ex}$ over the 100 cycles is close to the values shown in Table 2. These values are the magnitude of the $\mu_{ex}$ required to maintain 0.25 M of a solute in a bulk aqueous mixture devoid of any protein, and are approximately equal to the hydration free energy.

**Table 2**

| Fragment | HFE[fep 31] (kcal/mol) | $\mu_{ex}$ (kcal/mol) |
|---|---|---|
| Benzene | -0.71 | -0.79 |
| Propane | 1.60 | 1.96 |
| Acetaldehyde | -4.43 | -3.23 |
| Methanol | -6.16 | -5.62 |
| Formamide | -10.71 | -10.92 |
| Imidazole | -12.55 | -14.18 |
| Acetate | -96.5 | -97.31 |
| Methylammonium | -52.0 | -68.49 |
| Water | - | -5.6 |

Ten GCMC-MD simulations were run for each protein system. Each of these 10 simulations constituted 100 cycles of GCMC and MD (200 in the case of AR), with each cycle involving 100,000 steps of GCMC and 0.5 ns of MD, yielding a total of 100 million steps of GCMC and 500 ns of MD for every protein system (200 million steps of GCMC and 1 μs of MD for AR). Through the 100,000 steps of GCMC, both the solute molecules (fragments) and waters are exchanged between their gas-phase reservoirs and a sub-volume encapsulating the LBP of the protein. The configuration at the end of the GCMC is used as the starting configuration in the MD.

Before starting MD, a 500 step steep descent (SD) minimization and a 100 ps equilibration are run. The excess chemical potential ($\mu_{ex}$) supplied to drive solute and water exchanges during GCMC are periodically fluctuated over every 3 cycles, such that the average $\mu_{ex}$ over the 100 cycles is close to the values in Table 2. These values are the magnitude of the $\mu_{ex}$ required to maintain 0.25 M of a solute in a bulk aqueous mixture devoid of any protein, and are approximately equal to the hydration free energy. Since $\mu_{ex}$ is periodically fluctuated, throughout GCMC, the system is not a formal GC ensemble. However, by maintaining the average $\mu_{ex}$ constant over the length of the simulation the extent of deviation was minimal. For all proteins exemplified in this method, convergence of the FragMaps was monitored by calculating overlap coefficients (OC).

The ten trajectories were divided into two groups (group 1, trajectories 1-5; group 2, trajectories 6-10), and FragMaps of each group were separately computed. OC relates the overlap between FragMaps of two groups (group 1, trajectories 1-5 and group 2; trajectories 6-10) to a number between 0 and 1, with 1 reflecting completely identical maps. OC is calculated using equation (3).

$$OC = \sum_{i=1}^{N} \min \left( \frac{Q_i^i}{\sum_{j=1}^{N} Q_j^i} ; \frac{Q_i^2}{\sum_{j=1}^{N} Q_j^2} \right)$$

(3)

In equation 3 N is the number of voxels in the FragMaps and $Q_i^1$ and $Q_i^2$ are occupancies for the ith voxel from group 1 and 2 generated FragMaps, respectively. The ratios in the parentheses are computed to normalize the occupancy of each voxel by the sum of occupancies of all voxels in the corresponding FragMap. For each voxel, the smaller values (the conserved part) from group 1 and 2 are summed over all voxels to get the OC. It should be noted that the OC index does not behave linearly, such that a relatively small difference in the two distributions leads to a decrease from 1 to approximately 0.8, and values of >0.5 indicate a high degree of similarity (Fig. 11). As shown Table 3, reasonable overlap coefficients are recorded for FragMaps of all the tested systems.

**Table 3**

| Receptor | APOLAR | POS | NEG | HBDON | HBACC |
|----------|--------|------|------|-------|-------|
| AR | 0.68 | 0.65 | 0.63 | 0.41 | 0.47 |
| PPARγ | 0.78 | 0.66 | 0.75 | 0.58 | 0.63 |
| mGluR | 0.75 | 0.52 | 0.55 | 0.55 | 0.49 |
| B2A | 0.73 | 0.79 | 0.49 | 0.53 | 0.51 |
| B2I | 0.77 | 0.73 | 0.62 | 0.49 | 0.62 |

## VII. GCMC-only simulations

To probe the role of protein flexibility in modulating the FragMaps in the LBPs, a second set of GCMC-only simulations were run for all the protein systems. Like the GCMC/MD simulation, 10 independent runs were set up. Through the 100 cycles, no MD was run at the end of 100,000 steps of GCMC. However, the last configuration at the end of the 100,000 steps of GCMC was used as the input configuration for the next cycle. As with the GCMC-MD, $\mu_{ex}$ is periodically fluctuated over every 3 cycles around the values shown in Table 2, above.

## VIII. Protein Preparation

### AR & PPARγ:

PDB coordinates of 2AM9 (resolution 1.64 Å) and 3U9Q (resolution 1.52 Å) with the ligands testosterone (TES) and decanoic acid (DA), respectively, were used for the computer based methods of the present invention. Following the deletion of the ligands, missing residues 262-275 in the PDB 3U9Q were built using MODELLER. A total of 100 models were generated and ranked using the Discrete Optimized Protein Energy (DOPE) method and the highest ranking model was used as a starting structure. Crystal water molecules were retained, as were any structurally important ions. An in-house preparation script utilized GROMACS utilities to generate the simulation system involving protein, water, and small molecules, with the size of the system so as to have the protein extremity separated from the edge by 12 Å on all sides. The net system charge was made neutral by replacing random water molecules with the appropriate number of sodium or chloride ions. The proteins were minimized for 500 steps with the steepest descent (SD) algorithm in the presence of periodic boundary conditions (PBC). This was followed by a 100 ps equilibration during which temperature was adjusted by velocity rescaling. During the minimization and equilibration, harmonic positional restraints with a force constant of 2.4 kcal mol$^{-1}$ Å$^{-2}$ were applied to protein non-hydrogen atoms. The final coordinates at the end of equilibration are used as the starting conformations for the GCMC/MD simulation.

## IX. Virtual Screening (VS)

An *in silico* database of about 1.8 million compounds which contains all accessible tautomers and protonation states of each compound from the CHEMBRIDGE and MAYBRIDGE databases was used for screening. 1) Distinct B2A FragMap affinities (A1, A2, PI, P2, HBD1 and HBD2 in Fig. 12) were converted into pharmacophore features (SILCS-Pharm) using a method that is an extension to our recently published work. PHARMER was used to annotate each of the ligands from the database with the SILCS-PHARM features and then match these with the features from the FragMaps based on the RMSD of an alignment of the matches features. A cutoff of RMSD < 1.6 Å yielded 11,119 ligands. These ligands were docked into both the active (PDB:3P0G) and inactive (PDB:2RH1) structures of $\beta_2$AR using Autodock Vina, and differences in scores of the top ranked conformations were calculated as:

$$\Delta E_{dock} = \left| E_{dock}^{act} \right| - \left| E_{dock}^{inact} \right|$$

where $E_{dock}^{act}$ and $E_{dock}^{inact}$ are scores from top ranked poses in the active and inactive structures, respectively. A second selection criterion of $\Delta E_{dock} > 0$, yielded 906 ligands. LGFEs of these 906 ligands were calculated as Boltzmann averages over 1000 steps MC sampling in fields of both active (LGFEact) and inactive (LGFEinact) FragMaps. Differences in these LGFE scores were calculated as $\Delta LGFE = |LGFE^{act}| - |LGFE^{inact}|$, and 109 ligands were selected with $\Delta LGFE > 0$. Chemical fingerprint-based cluster analysis was used to select 15 chemically diverse ligands with which functional assessment studies were performed.

### *Ex vivo* intact Airway physiology:

All mouse studies were approved by the Animal Care and Use Committee of UMB. 5 mm sections of trachea from FVB/N mice were excised and studied in an isometric myograph system (ADInstrument) as described in the literature. A passive tension of 0.5 g was applied for each ring for a baseline. Rings were contracted with 1 μM acetylcholine, followed by the addition of 100 μM of isoproterenol (iso) and the selected ligands from VS studies. Percentage of relaxation in the presence of a ligand was measured as change in tension from acetylcholine-stimulated peak contraction. Rings were washed for reuse. Relaxation was calculated as an average over 9 runs with isoproterenol and 4 runs with each of the selected ligands.

## EXAMPLES

### I. Aqueous Solution Systems

For Scheme I and the aqueous mixture of Scheme II, the target concentration is obtained by varying $\mu_{ex}$ through the GCMC-MD iterations. To start, the value of $\mu_{ex}$ for both the solute and water is initially set to 0. In both cases, the average value of $\mu_{ex}$ for both solutes and water converged close to their HFE. Table 4 lists both the calculated and the experimental HFE (HFEexp) of the solutes. Average $\mu_{ex}$ of solute and water for the Scheme I and Scheme II GCMC-MD simulations with a spherical system of radius 25 Å were obtained from cycles 150-200. Averages and standard deviations were based on the 10 individual simulations of each system.

**Table 4**

| Fragment | HFEexp (kcal/mol) | HFEfep (kcal/mol) | Scheme I | | Scheme II | |
|---|---|---|---|---|---|---|
| | | | $\mu_{ex}$(kcal/mol) | Conc (M) | $\mu_{ex}$kcal/mol) | Conc (M) |
| Benzene | -0.83 | -0.86 | -0.71±0.37 | 1.40±0.11 | -0.94±0.13 | 0.32±0.19 |
| Propane | 1.96 | 1.89 | 1.35±0.12 | 1.37±0.26 | 1.34±0.52 | 0.36±0.11 |
| Acetaldehyde | -3.5 | -2.87 | -3.1±0.54 | 1.01±0.11 | -2.86±0.16 | 0.24±0.12 |
| Methanol | -5.1 | -4.83 | -5.79±0.23 | 1.3±0.49 | -4.92±0.14 | 0.22±0.08 |
| Formamide | -14 | -9.12 | -14.2±2.10 | 1.11±0.14 | -12.33±2.20 | 0.20±0.09 |
| Acetate | -79.1 | -97.43 | -48.1±0.54 | 0.82±0.21 | -52.1±0.49 | 0.24±0.03 |
| Meth. Amm | -71.3 | -60.02 | -58.1±0.34 | 0.71±0.23 | -56.1±0.52 | 0.22±0.13 |
| Water | -5.6 | - | -5.2+0.09 | 53.7±1.3 | -4.9±0.14 | 55.1±0.32 |

HFEs were also calculated using a fast energy perturbations (FEP) method, as previously described (see, Baker CM, et al., (2010), J Chem Theory Comput 6(4):1181-1198), to account for possible limitations in the force field that would yield a HFE in disagreement with the experimental data. Figure 2 traces the progression of both the concentration and $\mu_{ex}$ through the GCMC-MD iterations, as $\mu_{ex}$ was being varied based on $N_P^{target}$ in system A for both Scheme I and Scheme II. Both the concentration and the $\mu_{ex}$ at each GCMC-MD iteration in FIG. 2 are presented as the average over 10 independent simulations. In both Scheme I and Scheme II, the solutes and the solvent of system A attain their target $N_P^{target}$ values, corresponding to a concentration of 1 M and 0.25 M of solute in Scheme I and II, respectively, and a concentration of 55 M for water. For most of the systems, convergence occurred within 50 iterations. However, for the charged fragments the acceptance rates for particle insertions were low, due to the unfavorable electrostatic interactions. Because convergence took longer for these cases approximately 200 iterations of the simulation were needed to achieve convergence.

Table 4 lists the average $\mu_{ex}$ value and the concentration for each solute for 10 simulations, from the final 50 iterations. Because the average $\mu_{ex}$ value and concentration for solutes over the last 50 iterations are largely similar to corresponding $\mu_{ex}$ and concentration values measured across iterations 50-200 (Table 5) of the simulation, these results are consistent with the onset of convergence starting at iteration 50.

**Table 5**

| Fragment | Scheme I | | Scheme II | |
|---|---|---|---|---|
| | $\mu_{ex}$(kcal/mol) | Conc (M) | $\mu_{ex}$(kcal/mol) | Conc (M) |
| Benzene | -0.62±0.47 | 1.21±0.21 | -0.94±0.23 | 0.32±0.21 |
| Propane | 1.39±0.23 | 1.32±0.13 | 1.31±0.21 | 0.37±0.13 |
| Acetaldehyde | -3.1±0.71 | 1.1±0.81 | -2.92±0.43 | 0.24±0.12 |
| Methanol | -5.73±0.31 | 1.1±0.34 | -4.92±0.21 | 0.25±0.11 |
| Formamide | -14.2±1.22 | 1.03±0.43 | -11.51±1.31 | 0.22±0.12 |
| Acetate | -50.2±0.71 | 0.83±0.31 | -52.1±0.62 | 0.24±0.04 |
| Meth. Amm. | -58.1±0.42 | 0.71±0.32 | -56.1±0.74 | 0.22±0.17 |
| Water | -5.2+0.12 | 54.4±1.1 | -5.0±0.13 | 55.1±0.41 |

For hydrophobic and polar molecules, the average $\mu_{ex}$ values compare well with the HFE[fep]. The largest deviation occurs with formamide with the values falling between the HFE[exp] and HFE[fep] values. The deviation of $\mu_{ex}$ and HFE[fep] from the HFE[exp] for charged fragments, acetate and methylammonium, is due to the fact that the vacuum-to-solvent interface potential is not being accounted for in the present calculations. For monovalent anions/cations, this contribution was calculated to be about +/-12.5 kcal/mol, respectively, using the TIP3P water model. Further, as GCMC methods with charged solutes are limited by low acceptance rates for particle insertions, the present estimates of $\mu_{ex}$ for charged systems are within acceptable limits. Overall, these results establish that the presented iterative GCMC-MD methodology is useful towards estimating the HFE of organic solutes by virtue of their $\mu_{ex}$ in standard state aqueous systems. In addition, the approach is suitable for more complex aqueous mixtures as evidenced by the fact that the $\mu_{ex}$ values obtained from Scheme II are in satisfactory agreement with the experimental and HFE[fep] values.

While the GCMC-MD simulation protocol achieved the correct concentration and $\mu_{ex}$ in the system, the method was investigated for its ability to obtain the correct spatial sampling of solutes in finite spherical systems *e.g.,* heterogeneous systems. Spatial sampling was investigated via the analysis of radial distribution functions (RDF). RDFs of selected solute atoms and water oxygens were calculated from the cumulative MD sampling of both the Scheme I and Scheme II GCMC-MD simulations.

These RDF's were compared to the RDFs obtained from explicit-water 15 ns PBC-MD simulations. The PBC-MD simulations as described above maintain concentrations of solutes and water according to the parameters set forth for Scheme I and Scheme II in a cubic box whose sides are 50 Å each. These simulations also include an explicit treatment for long-range non-bonding interactions via particle mesh Ewald and isotropic LJ correction methods.

The RDFs from finite spherical systems GCMC-MD sampling and the explicit-water PBC MD simulations match very well. These results indicate that the use of oscillating $\mu_{ex}$ values to drive GCMC sampling and that the treatment of the long-range non-bonding interactions in the presented GCMD-MD protocol does not significantly impact the spatial sampling of the aqueous systems. Some fluctuations are seen in the RDF from Scheme II PBC, which are due to sampling issues, for instance, the MD only simulations were run for 15 ns versus a total of 100 ns MD simulations in the GCMC-MD protocol. Thus, the present methodology attains spatial sampling consistent with that observed in unbiased MD PBC simulations. It is noted that the $\mu_{ex}$ value settled close to the HFE values in a Scheme I simulation of acetaldehyde and methanol without MD at the end of every iteration. However, since the molecules are rigid during GCMC, MD simulations are likely needed to preserve the correct conformational and spatial sampling of the solutes in these bulk-phase environments.

Subsequent calculations focused on determining if the GCMC-MD approach could obtain equilibrium solute sampling with known, fixed $\mu_{ex}$ values. Accordingly, a set of GCMC-MD simulations were run, where instead of starting the simulation by assigning $\mu_{ex}$ to be 0, the method holds the value of $\mu_{ex}$ fixed to the HFE throughout the iterations. Shown in Figure 3 are the concentration and $\mu_{ex}$ for the acetaldehyde system as a function of the GCMC-MD iterations. In these simulations, it was found that as the number of iterations increased and waters attained bulk concentrations, the number of solute exchanges decreased considerably. Similar trends were seen for the other solutes (FIG. 8 and 9). Without ascribing to a specific theory, it is believed that the decrease in the number of solute exchanges is most likely the outcome of the cavity bias search used during GCMC moves. When the moves are performed for one molecule, the other molecules (both solutes and waters) are stationary and participate only in non-bonding interactions with the current molecule being exchanged, thereby preventing overlapping moves of molecules into locations already occupied. Thus, at the 55 M bulk-phase concentration of water, it is easier for the smaller water molecules to fill up cavities in system A than it is for the larger solutes, leading to a drastic decrease in the GCMC exchange probabilities for the solutes (FIG. 3, C and D). As continuous GCMC exchanges of fragments through insertions, deletions, and local relaxation through translations and rotations are important to maintain chemical equilibrium between system A and the coupled gas-phase fragment reservoirs the decrease in the number of solute exchanges was deemed to be problematic.

To overcome this, $\mu_{ex}$ of both the solutes and the water were cyclically fluctuated around their respective HFEs.

$$ d\mu_P = \frac{N_P}{N_P^{target}} $$

was alternately added and subtracted to $\mu_P$ (P=$F_1$, $F_2$, ..., $F_M$,W) over every three iterations of GCMC. Such variations in $\mu_{ex}$ lead to improvements in sampling over the course of the GCMC-MD iterations, as evidenced by both the change in the number of the solute molecules and increase in the GCMC move probabilities ($P_{ins+del}$ and $P_{trans+rot}$ for insertions, deletions, translations and rotation moves respectively), as shown in Figure 3. Similar trends were observed for the apolar and the other polar solutes (FIG. 8 and FIG. 9). Thus together with GCMC of both solvent and solute, it is important to continue to fluctuate the $\mu_{ex}$ supplied to these molecules once the target concentration, as described in equation 2, is attained to maintain efficient sampling of the solutes in the simulation system A.

## II. The T4 Lysozyme Mutant L99A System

T4-L99A, which contains an engineered occluded binding site for benzene, was selected as a model system. The GCMC-MD sampling method was identical to the aqueous systems above, except that system B was treated as periodic with the solute being studied included in system B at the target concentration used to drive the GCMC sampling. System A was a 20 Å sphere centered on the T4-L99A binding site defined by residues Ala 99 and Met 102 (FIG. 10).

Scheme I calculations on T4 involved benzene as the only solute at a concentration of 1 M along with water at a bulk concentration of 55 M. These parametric constraints permitted validation of the method, namely to drive sampling of benzene in the occluded binding pocket of the protein as well as yield a quantitative estimate of ligand binding. Across a 10×37.5 ns GCMC-MD simulation with conformations saved every 10 ps, the method showed that benzene was bound to the T4-L99A binding site for a total of 372 ns with the pocket being empty for only 3 ns. The average benzene concentration in the simulation system A was 1.5±0.2 M. These results indicate that the GCMC-MD method can sample the occluded pocket as well as attain the defined concentration in the entire system that drives the GCMC sampling.

The pocket sampling also permitted the estimation of the binding affinity of a ligand ΔG°, using the following equation:

$$ \Delta G^\circ = -RT \ln \frac{[PL]}{[P]} + RT \ln [L] V_{ref} $$

$$ (4) $$

In equation (4), R is the gas constant (kcal/mol/K), T (K) is the temperature, [PL] is the concentration of the bound ligand, [L] is the total concentration of the ligand, [P] is the concentration of the protein, $V_{ref}$ is the reference volume in concentration

units (-1660 Å$^3$ per one ligand molecule for 1 M of ligand). Since the simulations are maintained in equilibrium, the bound vs. unbound ligand concentrations can be correlated to the time fraction of ligand bound vs. unbound in the binding site through the simulation. As the T4-L99A pocket is completely occluded, the presence or the absence of a solute atom at the active site is driven only by the GCMC insertion/deletion moves. Over the 10×37.5 ns GCMC-MD simulation that involves a total of 18.75 million GCMC insertion/deletion attempts for the benzene, with [L] ~ 1.5 M and a [PL]/[P] ratio of 99.4/0.6, ΔG°, calculated using Equation 2, is about -3.25 kcal/mol. Although there is some difference from the experimental binding affinity of - 5.19±0.16 kcal/mol, it should be noted that an increase of 10$^4$ steps out of a total of 1.9 x10$^7$ steps with benzene in the pocket, translates to nearly 1 kcal/mol difference in the calculated ΔG°. This does not mean a lack of equilibrium in the system, but emphasizes the difficulty of converging the calculation of a binding constant to a single site in a protein, although force field effects could also impact the obtained ΔG°.

Scheme II calculations included seven solutes along with water. As with the aqueous system, the target concentration for the solutions of each solute was 0.25 M. This simulation was run for a total of 10×50 ns. To facilitate analysis of the results, affinity patterns of selected atoms from different solutes in the occluded binding pocket, called "Grid Free Energy (GFE) FragMaps", were calculated.

GFE FragMaps are Boltzmann transformed probability distributions for the solute atoms that are normalized using the distributions of solute molecules in an aqueous solution that does not contain the macromolecule. This normalization accounts for the free energy penalty due to solute desolvation when calculating the GFEs. These maps may then be visualized to qualitatively evaluate the ability of the GCMC-MD sampling method to reproduce the positions of different ligands known to bind T4-L99A that have been subjected to experimental analysis. Nine ligands were considered: 1) benzene, 2) o-xylene, 3) p-xylene, 4) ethylbenzene, 5) benzofuran, 6) indene, 7) indole, 8) isobutylbenzene, and 9) n-butylbenzene.

FIG. 4 shows the aromatic (BENC), aliphatic (PRPC), polar hydrogen bond donors (MEOH, FORH) and acceptors (MEOO, FORO, AALO) maps along with the crystallographic orientations of the ligands. Benzene occupies the aromatic FragMap while the aliphatic moieties of the other ligands occupy the aliphatic FragMap region that protrudes away from the benzene molecule. In addition, polar H-bond donor and acceptor maps are in the binding pocket and are in the vicinity of the corresponding functional groups on benzofuran and indole. Importantly, although the protein structure from the T4-L99A-benzene complex (PDB 181L) are used as the starting conformation, GCMC-MD simulations correctly identify the ability of the pocket to alter its conformation to allow favorable interaction with the aliphatic moieties as well as its ability to accommodate polar functionality. The ability of the inventive method to accommodate protein flexibility is due to the application of MD, which accounts for alterations in protein conformation (protein flexibility) during binding.

The use of GFE FragMaps has other advantages too. For instance, GFE FragMaps permits quantitative evaluation of the relative affinity of ligands, based on Ligand GFE (LGFE) scores, as described above.

LGFE scores quantify the overlap of atoms in the ligand with the corresponding GFE FragMaps. LGFE scores were calculated as Boltzmann weighted averages from ensembles of ligand-protein orientations generated using i) MD sampling of the ligands bound to the protein and ii) MC sampling of the ligands in the field of FragMaps. As shown in FIG. 5, both LGFE$^{MD}$ and LGFE$^{MC}$ correlate very well with the experimental binding free energies (high R$^2$ and predictive index (PI). Importantly, the LGFEs can distinguish between the binding activity of both congeneric series and diverse classes of ligands. The range of experimental binding affinities is -2.1 kcal/mol while the LGFE scores from the protein+ligand MD ensemble and the MC sampling are spread over wider ranges of -4.42 and -6.1 kcal/mol, respectively. This is not unexpected as the LGFE scores not true free energies of binding as numerous terms that contribute binding are omitted (eg. the configurational entropy of the ligands).

### III. Mapping of Functional Group Free Energy Patterns at Occluded Sites of Proteins

Eight representative solutes with different chemical functionalities: benzene, propane, acetaldehyde, methanol, formamide, imidazole, acetate, and methylammonium were chosen to probe the LBPs. Benzene and propane serve as probes for nonpolar functionalities, while methanol, formamide, imidazole and acetaldehyde are neutral molecules that participate in hydrogen bonding. The positively charged methylammonium and negatively charged acetate molecules serve as probes for charged donor and acceptor groups, respectively. The normalized probability distributions for selected atoms in these solutes from the SILCS-GCMC/MD simulations were then used to create functional group affinity FragMaps at the respective LBPs as described below.

### a. Androgen Receptor

To create FragMaps, a SILCS-GCMC/MD was run with the testosterone-AR crystal structure (PDB 2AM9) after the removal of testosterone. Although the simulations were initiated with the steroid-bound conformation of AR, FragMaps from the 10x100ns GCMC/MD simulations recapitulate the locations of different functional groups for both steroidal and non-steroidal crystallographic ligands (Fig. 13). Thus, cycloalkane rings of the steroidal ligands that occupy the largely hydrophobic pocket of the AR shown to have good overlap with the apolar (APOLAR) FragMaps (A1 in Fig. 13A). The ketone groups of TES and dihydrotestosterone (DHT) that hydrogen bond with R752 accordingly, overlap with the negatively charged (NEG) FragMaps (N1 in Fig. 13A). Similarly, hydrogen bond donor (HBDON) and positively charged (POS) FragMaps close to N705 overlap with the 17β-hydroxyl groups of the steroids.

Notably, the apolar cavity between the W741, L873 and T877 that is occupied by some ligands such as the steroidal EM-5744 and the non-steroidal S-1, an analog of R-bicalutamide, is inaccessible in the starting conformation. However, following simulation of the structure, the side chains of W741 and T877 undergo conformational changes that lead to the formation of a cavity without significantly affecting the global conformation of the androgen receptor. For instance, the root mean square deviation (RMSD) in the proteins (backbone is -1.2 Å, consequently, APOLAR FragMaps were found in this cavity (A2, Fig. 13A). As illustrated in Figure 13D, no APOLAR FragMaps densities were found in a GCMC-only simulation in which the protein was rigid. This comparison, validates that protein flexibility through the inclusion of MD is important since it permits solutes to sample regions of the protein that were unavailable in the starting conformation. Additional evidence of the importance of including protein flexibility was the notable increase in the area sampled by APOLAR FragMaps at the A1 site in the GCMC/MD vs. the GCMC-only simulation (Figs. 13A v/s 13D). The inventors believe this increase in sampling to be driven by the flexibilities of the side chains of residues that form the pocket. Taken together these results point to the qualitative ability of the SILCS-GCMC/MD approach to map the functional group requirements of a fully occluded LBP, including the ability of the SILCS method to identify regions accessible to solutes significantly beyond those present in the crystal structure.

***b. PPARγ:***

To map the functional group free energy patterns at the LBP of PPARγ a 10x50 ns SILCS-GCMC/MD was initiated using the PDB structure 3U9Q following removal of decanoic acid from the crystal structure. As illustrated in Figure14A, the FragMaps sample the decanoic acid pocket (marked LBP1 in Fig. 14A), and a second pocket that is flanked between helices H3, H4 and β-sheets B2 and B3 (LBP2). Between the two pockets, the LBP1 pocket is more occluded than LBP2 pocket (see, Table 6) and therefore, some sampling of LBP2 occurs even with the rigid protein conformation in the GCMC-only simulations. However, there is an increase in the extent of the sampling of FragMaps when protein flexibility is included via MD.

**Table 6.** Occludedness of a ligand binding pocket (LBP) measured as a ratio of the solvent accessible surface area (SASA) of a ligand in bulk vs. in the LBP.

| Receptor | Ligand | Bulk SASA | LBP SASA | Ratio |
|---|---|---|---|---|
| AR | Testosterone | 402.1 | 0 | 0 |
| PPARγ | Rosiglitazone | 506.8 | 68.6 | 0.13 |
| | Decanoic acid | 341.1 | 28.8 | 0.08 |
| mGluR | FITM | 576.8 | 43.8 | 0.07 |
| β₂AR | BI-167107 | 534.8 | 36.32 | 0.07 |
| | Carazalol | 478.6 | 62.8 | 0.13 |

In addition, as shown in Figure 15, the FragMaps trace a pathway from the protein surface to the LBP, indicating a possible pathway for ligand binding.

Further validation of the method is provided by greater extent of overlap between the terminal alkyl chain of decanoic acid and the APOLAR FragMap densities as well as the overlap between the carboxylic acid of decanoic acid and the NEG FragMap densities (A1, N1, respectively) in the LBP1 (Fig. 14A). Different functional groups of Rosiglitazone, a known antidiabetic drug that binds to the PPARγ in the LBP2 pocket was also found to overlap well with the FragMaps (Fig. 14). For instance, the thiazolidinedione moiety overlaps with the NEG FragMaps in the proximity of H323 and H449 (N1 in Fig. 14B), while the pyridine moiety overlaps with APOLAR FragMaps (A3) in the proximity of M364 and V339 and the ethoxy linker between the thiazolidinedione and the pyridine overlaps with HBACC FragMaps (HBA1).

Along with these qualitative observations, binding affinities of the ligands were estimated using Ligand Grid Free Energy (LGFE) scoring for 16 ligands whose binding activity data to the human PPARγ is available, and compared against the experimental binding affinity, $\Delta G_{bind}$. $K_I$s obtained from the different sources were normalized against the $K_I$ of rosiglitazone ($K_I$=120nM; binding DB reported a range of values between 8-440 nM). Despite the diversity in the ligands and their binding modes, there is reasonable correlation between the LGFE and $\Delta G_{bind}$ values with a predictive index (PI) of ~ 0.63 and $R^2$ -0.22. For instance, the compound GW409544 that binds both the LBP1 and LBP2 (PDB:1K74) pockets has very good overlap with APOLAR FragMaps A1, A2 and A3 (Fig. 14C), leading to a favorable LGFE that correlates with its high binding affinity compared to the partial agonist decanoic acid, or the thiazolidinediones such as the rosiglitazone. On the other hand, poor correlations are noted for Cerco-A (Fig. 14D) driven by a lack of APOLAR FragMaps in the hydrophobic cavity between L262 and F287, where the dibenzofurancaboxamide functional group of Cerco-A binds. The present inventors have hypothesized that the poor correlation is due to the loss of the hydrophobic cavity due to 1) the high flexibilities of the side chains of these residues during simulations and 2) the conformation of the loop connecting helices H2 and H3, modelled using MODELLER as described above. Although some of the side chains are flexible, the overall conformation of the receptor, however is preserved. In fact, LGFE of ligands calculated using FragMaps from the GCMC-only simulations correlate more poorly with the experimental $\Delta G_{bind}$. Since SBDD procedures typically screen ligands based on an estimation of their binding affinities, this result further validates the importance of incorporating protein flexibility in such studies.

### c. mGluR:

To efficiently sample the partially occluded LBP of mGluR1, 10x50 ns SILCS-GCMC/MD was performed on a monomer of mGluR obtained from a crystal structure of the inactive conformation of the dimeric 7 transmembrane (TM) region of the family C mGluR1 in complex with FITM, a negative allosteric modulator (NAM). During this sampling, the GCMC of solutes and water were restricted to a 20 Å cubic region around the LBP. FragMaps correctly recapitulate the different functional groups of the FITM (Fig. 16). The largely hydrophobic nature of the pocket is traced by the high affinities of the APOLAR FragMaps. Two distinct APOLAR densities overlap well with the pyrimidine amine and the p-fluorophenyl moieties of FITM, which are found in the neighborhood of V753, V664 and 1812 (A1 in Fig. 16A) and F801, 1797, W798 and L757 (A2), respectively. HBDON FragMaps in the proximity of T815, noted to be important for binding, overlap well with the pyrimidine of FITM. Although some of these side chains are found to be flexible through the GCMC/MD simulation, the overall conformation of the receptor, with the narrow binding pocket is preserved .

Mutational studies of the mGluR1 and binding activity with other allosteric modulators such as the 2-Methyl-6-(phenylethynyl)pyridine (MPEP) (another known NAM) revealed that the binding pocket identified with FITM could be shared with other NAMs. MC sampling of MPEP in the pocket and in the presence of FragMaps as described above yields binding modes similar to FITM (Fig. 16). LGFEs for the FITM, MPEP and other analogs of FITM correlated well with their $\Delta G_{bind}$ (SI Fig. 17C). Although some sampling of the pocket occurs through a GCMC-only simulation (Fig. 17A vs. 17B), similar to PPARγ the correlations were poor when LGFE scores were calculated using FragMaps from a GCMC-only simulation of mGluR (Fig. S17C vs. 17D).

### d. $\beta_2$AR:

Crystal structures of the inactive and the active conformations of the family A GPCR are available and two separate 10x50 ns SILCS-GCMC/MD were run with each structure. In this document, B2I refers to the simulations starting with the inactive conformation (PDB: 2RH1) and B2A refers to simulations starting with the active conformation (PDB: 3P0G).

Good overlaps were obtained between the FragMaps and the crystallographic ligands for both B2I and B2A. APOLAR FragMap affinities close to the hydrophobic region defined by F289, V117 and A200 overlapped well with the benzoxazine and carbazol moieties of BI161707 and with carazolol, respectively (A1 in Fig. 12). Both POS and HBDON FragMaps adjacent to D113 overlapped with the amine functional groups in both the ligands (DN2 and D2 in Fig. 12A). While the FragMaps recapitulate the location of respective ligands for both B2I and B2A, clear differences were found between the FragMaps for these two conformations.

A second APOLAR density was found close to the second hydrophobic pocket adjacent to W109, 1309, and F193 only in B2A (A2, Fig. 12A). The lack of this hydrophobic pocket in B2I is due to the relatively high flexibility of the 1309 and F193 side chains in B2I as compared to B2A (SI Fig.18A). Despite these side-chain flexibilities, the overall active and inactive conformations of $\beta_2$AR were retained (Fig. S18B). Mutational studies have shown that agonist interactions with 1309 are important for both $\beta_2$AR selectivity and ligand activation. Extensive interactions between the carbonyl oxygen, amine and the hydroxyl groups of BI-167101 with S203, S204 and S207 in B2A were correctly recapitulated by HBDON and HBACC FragMaps, while the lone polar nitrogen of the carbazol heterocycle in carazolol was recapitulated by narrower HBDON maps in B2I (HBD1, Fig. 12A). These differences may be attributed to the higher flexibilities of the S203 and S204 side chains in B2I (SI Fig. S18A). Consistent with these differential FragMaps are mutational studies that have identified the S203 and S204 to be important for agonist binding and receptor activation through their catecholamine hydroxyls.

Notably, the SILCS-GCMC/MD approach is able to quantitatively differentiate between the two states of $\beta_2$AR as well. LGFE scores from MC sampling were obtained for a diverse range of agonists, partial agonists and antagonists/inverse agonists. As shown in Table 7, good correlations were obtained between the LGFE scores and binding affinities of agonists and partial agonists with B2A ($R^2 \sim 0.46$, PI $\sim 0.59$, Fig. 12B-(1)) while the same set of ligands yield significantly worse correlations with the B2I FragMaps ($R^2 \sim 0.10$, PI $\sim 0.31$, Fig. 12B-(2)). Similarly, binding affinities of antagonists/inverse agonists correlated well with the LGFEs scored using B2I FragMaps ($R^2 \sim 0.45$, PI $\sim 0.67$, Fig. 12B-(4)), while poorer correlation was found with the LGFEs calculated using the B2A FragMaps ($R^2 \sim 0.11$, PI-0.38, Fig. 12B-(3)). Consistent with the quality of the correlations, MC sampling of ligands yielded binding modes similar to the crystallographic BI167107 and carazolol orientations (Fig. 19 & 20).

**FragMaps guided ligand screening for $\beta_2$AR:**

$\beta_2$AR are expressed on numerous cell types, including airway smooth muscle (ASM). Activation of $\beta_2$AR in ASM causes bronchodilation and inhaled beta-agonists are the main therapy for asthma and other obstructive pulmonary diseases. Differences in FragMaps between the two end states were used to guide virtual screening (VS) studies to identify new agonists for $\beta_2$AR. Following the procedure described above, 15 top scoring chemically diverse ligands (Fig. 21) were selected from an *in silico* database containing about 1.8 million compounds. Effect of the selected compounds were studied through a relaxation response of tracheal rings from mice lung samples. This *ex vivo* method is a relatively high-throughput strategy and a better predictor of *in vivo* macromolecular disposition than *in vitro* studies. Isoproterenol was used as positive control. Seven of these ligands effected varying degrees of tracheal relaxation (Fig. 12C), representing a 46% hit rate. Docked conformations of the selected ligands had good overlaps with the B2A FragMaps leading to high LGFE scores (Fig. 22). All the ligands occupied the hydrophobic cavity defined by residues 1309, W109 and F193 and maintained interactions with Asp 113. Ligands that caused tracheal relaxation were also seen to maintain interactions with the S203 and S207 in helix H5 identified to be important for ligand activation and $\beta_2$AR selectivity. These results point to the utility of the SILCS-GCMC/MD methodology in rational ligand design, including identification of agonists.

**Table 7**

|  | PDB | Compound | Function | $\Delta G_{bind}$ | Reference |
|---|---|---|---|---|---|
| 1 |  | Salbutamol | Agonist/ Partial Agonist | -8.09 | 46 |
| 2 | 4LDO | Adrenaline(epinephrine) |  | -8.60 | 47 |
| 3 |  | Isoproterenol |  | -9.63 | 47 |
| 4 | 4LDL | Hydroxybenzyl isoproterenol |  | -9.72 | 48 |
| 5 |  | Indacaterol |  | -10.19 | 49 |
| 6 |  | Formoterol |  | -10.60 | 49 |
| 7 |  | THRX-144877(AA1) |  | -11.04 | 49 |
| 8 |  | Picumeterol |  | -12.23 | 49 |
| 9 |  | Salmeterol |  | -12.00 | 50 |
| 10 | 3P0G | BI-167107 |  | -13.69 | 21 |
| 11 |  | Practolol | Antagonists/ inverse agonist | -6.77 | 50 |
| 12 |  | Atenolol |  | -8.14 | 50 |
| 13 |  | Acebutalol |  | -8.26 | 50 |
| 14 |  | Bisoprolol |  | -9.10 | 50 |
| 15 |  | Labetolol |  | -10.91 | 50 |
| 16 |  | Propranolol |  | -12.34 | 50 |
| 17 |  | Pindolol |  | -12.43 | 51 |
| 18 |  | ICI-118551 |  | -12.58 | 50 |
| 19 |  | Carvedilol |  | -12.77 | 50 |
| 20 | 3D4S | Timolol |  | -13.15 | 50 |
| 21 | 2RH1 | Carazolol |  | -13.35 | 52 |

**Claims**

1. A computational method for sampling the spatial distribution of one or more solutes and water in a defined region of space (system) comprising:

    1) assigning a target concentration, $N_{tgt}$, of each of the one or more solutes and water;
    2) sampling the spatial distribution of the one or more solutes and water in a computationally defined region of space using Grand-Canonical Monte-Carlo (GCMC) Metropolis sampling criteria, wherein the excess chemical potential ($\mu_{ex}$) assigned for each of the one or more solutes, if present, and water is set to 0, if present;
    3) updating $\mu_{ex}$ of each of the one or more solutes and water from the difference in current concentration in the defined region of space ($N_{sys}$) and the target ($N_{tgt}$) ;
    4) repeating steps 2) and 3) using the updated values of $\mu_{ex}$ in step 2) to obtain a spatial distribution of the one or more solutes and water;

    wherein the system further comprises one or more macromolecules, wherein the spatial distribution of one or more solutes or water is used to identify preferential affinity of each of the solutes or water to each of the one or more macromolecules, and wherein said one or more macromolecules are selected from a protein, RNA, DNA, carbohydrate, lipid, organic chemical, inorganic chemical, or a combination thereof.

2. The method according to claim 1 wherein the system contains water and one solute and where the target concentration of the solute and water is set at 1 M and 55 M, respectively, from which the hydration free energy of the solute is obtained from the value of $\mu_{ex}$

3. The method according to claim 1, wherein the $\mu_{ex}$ of one or more solutes and water is alternately increased and decreased during the GCMC operations across consecutive cycles involving steps 2)-4), after the concentration of the solutes and the water reach their target value.

4. The method according to claim 1, wherein, the proportion of GCMC steps for each of the one or more solutes and water is assigned based on the target concentration of each of the solutes and water.

5. The method according to claim 1, wherein the system containing the solutes and or water is encompassed in a larger system containing said solutes and or water.

6. The method according to claim 5, wherein the system additionally includes one or more macromolecules.

7. The method according to claim 1, wherein the spatial distribution following step 2) is sampled with a molecular dynamics simulation.

8. The method according to claim 1, wherein $\mu_{ex}$ is increased or decreased equal to $N_{tgt}/N_{sys}$.

9. The method according to claim 1, wherein $\mu_{ex}$ is increased when $N_{sys}$ is lower than the $N_{tgt}$ and decreased when $N_{sys}$ is greater than $N_{tar}$.

10. The method according to claim 1, wherein said computationally defined region comprises an inner region containing the one or more solutes and water, located within a larger outer region containing additional water.

11. The method according to claim 1, wherein an output of the spatial distributions of the one or more solutes and water is generated.

**Patentansprüche**

1. Rechenverfahren zum Beproben ("sampling") der räumlichen Verteilung eines oder mehrerer gelöster Stoffe ("solutes") und von Wasser in einer definierten Region eines Raum(system)s, umfassend:

    1) Zuweisen einer Zielkonzentration, $N_{tgt}$, zu jedem des einen oder der mehreren gelösten Stoffe und Wasser;
    2) Beproben der räumlichen Verteilung des einen oder der mehreren gelösten Stoffe und von Wasser in einer rechnerisch definierten Raumregion unter Verwendung großer kanonischer Metropolis-Monte-Carlo (Grand-Canonical Monte-Carlo; GCMC)-Beprobungskriterien, wobei das für jeden des einen oder der mehreren gelösten Stoffe zugewiesene, falls vorhanden, und für Wasser zugewiesene überschüssige chemische Potential ($\mu_{ex}$) auf 0 eingestellt wird, falls vorhanden;

3) Aktualisieren von $\mu_{ex}$ von jedem des einen oder der mehreren gelösten Stoffe und Wasser aus der Differenz der aktuellen Konzentration in der definierten Raumregion ($N_{sys}$) und dem Ziel ($N_{tgt}$);

4) Wiederholen der Schritte 2) und 3) unter Verwendung der aktualisierten Werte von $\mu_{ex}$ in Schritt 2), um eine räumliche Verteilung des einen oder der mehreren gelösten Stoffe und von Wasser zu erhalten;

wobei das System weiterhin ein oder mehrere Makromoleküle umfasst, wobei die räumliche Verteilung eines oder mehrerer gelöster Stoffe und von Wasser verwendet wird, um die präferentielle Affinität jedes der gelösten Stoffe oder von Wasser zu jedem des einen oder der mehreren Makromoleküle zu identifizieren, und wobei das eine oder die mehreren Makromoleküle aus einem Protein, RNA, DNA, Kohlenhydrat, Lipid, organischer Chemikalie, anorganischer Chemikalie oder einer Kombination davon ausgewählt sind.

2. Verfahren gemäß Anspruch 1, wobei das System Wasser und einen gelösten Stoff enthält und wobei die Zielkonzentration des gelösten Stoffs und des Wassers auf 1 M bzw. 55 M eingestellt wird, woraus die freie Hydratationsenergie des gelösten Stoffs aus dem Wert von $\mu_{ex}$ erhalten wird.

3. Verfahren gemäß Anspruch 1, wobei der $\mu_{ex}$ eines oder mehrerer gelöster Stoffe und von Wasser während der GCMC-Operationen über aufeinanderfolgende Zyklen, die die Schritte 2)-4) involvieren, abwechselnd erhöht und verringert wird, nachdem die Konzentration der gelösten Stoffe und des Wassers ihren Zielwert erreicht.

4. Verfahren gemäß Anspruch 1, wobei der Anteil an GCMC-Schritten für jeden des einen oder der mehreren gelösten Stoffe und von Wasser auf Grundlage der Zielkonzentration jedes der gelösten Stoffe und von Wasser zugewiesen wird.

5. Verfahren gemäß Anspruch 1, wobei das die gelösten Stoffe und oder Wasser enthaltende System in einem größeren System umfasst ist, welches die gelösten Stoffe und oder das Wasser enthält.

6. Verfahren gemäß Anspruch 5, wobei das System zusätzlich ein oder mehrere Makromoleküle einschließt.

7. Verfahren gemäß Anspruch 1, wobei die räumliche Verteilung nach Schritt 2) mit einer Molekular-Dynamik-Simulation beprobt wird.

8. Verfahren gemäß Anspruch 1, wobei $\mu_{ex}$ gleich $N_{tgt}/N_{sys}$ erhöht oder verringert wird.

9. Verfahren nach Anspruch 1, wobei $\mu_{ex}$ erhöht wird, wenn $N_{sys}$ niedriger als die $N_{tgt}$ ist, und verringert wird, wenn $N_{sys}$ größer als $N_{tar}$ ist.

10. Verfahren gemäß Anspruch 1, wobei die rechnerisch definierte Region eine innere Region umfasst, welche den einen oder die mehreren gelösten Stoffe und Wasser enthält, lokalisiert innerhalb einer größeren äußeren Region, die zusätzliches Wasser enthält.

11. Verfahren gemäß Anspruch 1, wobei ein Ausgabewert der räumlichen Verteilungen des einen oder der mehreren gelösten Stoffe und von Wasser erzeugt wird.

**Revendications**

1. Procédé informatisé pour échantillonner la distribution spatiale d'un ou plusieurs solutés et d'eau dans une région d'espace définie (système) comprenant les étapes consistant à :

1) attribuer une concentration cible, $N_{tgt}$, de chacun des un ou plusieurs solutés et de l'eau ;

2) échantillonner la distribution spatiale des un ou plusieurs solutés et de l'eau dans une région d'espace définie informatiquement en utilisant des critères d'échantillonnage Metropolis Monte-Carlo à ensemble grand canonique (GCMC), dans lequel le potentiel chimique en excès ($\mu_{ex}$) attribué à chacun des un ou plusieurs solutés, le cas échéant, et à l'eau est réglé sur 0, le cas échéant ;

3) mettre à jour le $\mu_{ex}$ de chacun des un ou plusieurs solutés et de l'eau à partir de la différence de concentration de courant dans la région d'espace définie ($N_{sys}$) et la cible ($N_{tgt}$) ;
4) répéter les étapes 2) et 3) en utilisant les valeurs mises à jour de $\mu_{ex}$ à l'étape 2) pour obtenir une distribution spatiale des un ou plusieurs solutés et de l'eau ;

dans lequel le système comprend en outre une ou plusieurs macromolécules, dans lequel la distribution spatiale d'un ou plusieurs solutés ou d'eau est utilisée pour identifier une affinité préférentielle de chacun des solutés ou de l'eau pour chacune des une ou plusieurs macromolécules, et dans lequel lesdites une ou plusieurs macromolécules sont sélectionnées parmi une protéine, un ARN, un ADN, un glucide, un lipide, un produit chimique organique, un produit chimique inorganique, ou une combinaison de ceux-ci.

2. Procédé selon la revendication 1, dans lequel le système contient de l'eau et un soluté et où la concentration cible du soluté et de l'eau est fixée à 1 M et 55 M, respectivement, à partir desquelles l'énergie libre d'hydratation du soluté est obtenue à partir de la valeur de $\mu_{ex}$.

3. Procédé selon la revendication 1, dans lequel le $\mu_{ex}$ d'un ou plusieurs solutés et d'eau est augmenté et diminué alternativement au cours des opérations de GCMC pendant des cycles consécutifs impliquant les étapes 2) à 4), une fois que la concentration des solutés et de l'eau ont atteint leur valeur cible.

4. Procédé selon la revendication 1, dans lequel la proportion d'étapes de GCMC pour chacun des un ou plusieurs solutés et de l'eau est attribuée en fonction de la concentration cible de chacun des solutés et de l'eau.

5. Procédé selon la revendication 1, dans lequel le système contenant les solutés et/ou de l'eau est inclus dans un système plus grand contenant lesdits solutés et/ou l'eau.

6. Procédé selon la revendication 5, dans lequel le système inclut en plus une ou plusieurs macromolécules.

7. Procédé selon la revendication 1, dans lequel la distribution spatiale suivant l'étape 2) est échantillonnée avec une simulation de dynamique moléculaire.

8. Procédé selon la revendication 1, dans lequel $\mu_{ex}$ est augmenté ou diminué égal à $N_{tgt}/N_{sys}$.

9. Procédé selon la revendication 1, dans lequel $\mu_{ex}$ est augmenté lorsque $N_{sys}$ est inférieure à $N_{tgt}$ et diminué lorsque $N_{sys}$ est supérieure à $N_{tar}$.

10. Procédé selon la revendication 1, dans lequel ladite région définie informatiquement comprend une région interne contenant les un ou plusieurs solutés et de l'eau, située dans une région externe plus grande contenant de l'eau supplémentaire.

11. Procédé selon la revendication 1, dans lequel une sortie des distributions spatiales des un ou plusieurs solutés et de l'eau est générée.

SCHEME I

SCHEME II

FIG. 1

FIG. 2

FIG. 2

CONT.

**FIG. 3A**

**FIG. 3B**

**FIG. 3C**

**FIG. 3D**

*FIG. 4*

**FIG. 5**

**FIG. 6A**

**FIG. 6B**

EP 3 100 023 B1

FIG. 7

FIG. 8

**FIG. 9**

FIG. 10

FIG. 11

FIG. 12A

EP 3 100 023 B1

FIG. 12C

FIG. 12D

FIG. 12B

GCMC/MD

FIG. 13A    FIG. 13B

FIG. 13C    FIG. 13D

GCMC-ONLY

TES

S-1

EM-5744

DECANOIC ACID

**FIG. 14A**

ROSIGLITAZONE

**FIG. 14B**

GW409544

**FIG. 14C**

CERCO-A

**FIG. 14D**

**FIG. 15**

**FIG. 16A**

**FIG. 16B**

GCMC/MD

**FIG. 17A**

GCMC-ONLY

**FIG. 17B**

**FIG. 17C**

**FIG. 17D**

FIG. 18B

FIG. 18A

*FIG. 19*

**FIG. 20**

FIG. 21

FIG. 22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 107331

**Non-patent literature cited in the description**

- **DILL KA ; BROMBERG S.** *Chemistry and Biology,* 2003
- **DENG Y ; ROUX B.** *J Chem Phys.,* 21 March 2008, vol. 128 (11), 115103
- **CLARK M et al.** *J Chem Inf Model.,* April 2009, vol. 49 (4), 934-43
- **MORTON A ; BAASE WA ; MATTHEWS BW.** *Biochemistry,* 1995, vol. 34 (27), 8564-8575
- **CALEMAN C et al.** *Proc Natl Acad Sci USA,* 2011, vol. 108 (17), 6838-6842
- **BAKER CM et al.** *J Chem Theory Comput,* 2010, vol. 6 (4), 1181-1198